# EUROPEAN PATENT SPECIFICATION

(11) **EP 4 518 955 B1**
(45) Date of publication and mention of the grant of the patent: **18.06.2025**
(21) Application number: 23724798.6
(22) Date of filing: 04.05.2023
(51) Int. Cl.: A61N 1/05, A61B 5/00, A61B 5/293

(54) **IMPLANTABLE ELECTRICAL/ELECTRONIC BIOMEDICAL DEVICE.**
IMPLANTIERBARE ELEKTRISCHE/ELEKTRONISCHE BIOMEDIZINISCHE VORRICHTUNG.
DISPOSITIF BIOMÉDICAL ÉLECTRIQUE/ÉLECTRONIQUE IMPLANTABLE.

(30) Priority: 06.05.2022 EP 22172164
(43) Date of publication of application: 12.03.2025
(73) Proprietor: ECOLE POLYTECHNIQUE FEDERALE DE LAUSANNE (EPFL), 1015 Lausanne (CH)
(72) Inventor: SONG, Sukho, Ansan Gyeonggi-do 15651 (KR); VACHICOURAS, Nicolas, 1292 Chambesy (CH); SEREX, Ludovic, 1003 Lausanne (CH); FALLEGGER, Florian, 1007 Lausanne (CH); HUGUET, Benoit, 1022 Chavannes-Renens (CH); LACOUR, Stephanie P., 1163 Etoy (CH)
(74) Representative: M. Zardi & Co S.A.
(86) International application number: PCT/EP2023/061860
(87) International publication number: WO 2023/213974

(56) References cited:
- WO-A1-2021/005382
- US-A1- 2006 129 203
- US-B1- 6 368 349

## Description

### Filed of application

The present invention relates to an implantable electrical/electronic biomedical device, for instance for use in an electrocorticography (ECoG).

The present invention also relates to a system including the implantable electrical/electronic biomedical device and to a method to use the system and the implantable electrical/electronic biomedical device.

### Prior art

In the following description, prior art references are cited by means of Arabic numerals (from 1 to 63) in parentheses, and all numerals are reported at the end of the prior art section, along with the details of the corresponding refences, such as the author, the title and date of publication. This presentation of information is given for clarity.

Implantable electrical/electronic biomedical devices are known, for instance for use in Electrocorticography (ECoG).

Electrocorticography (ECoG) grids are a class of surface electrode implants used to investigate neural activities across large areas of the brain (*1-4*). ECoG grids find a wide variety of applications in basic neuroscience and clinical settings including brain function monitoring (*7-10*), alleviation of epileptic seizure (*11*), brain function recovery (*12*, *13*), pain modulation (*14*), speech recognition (*15*) and brain-computer interfaces (BCI) (*16*).

The large area coverage of ECoG grids is also useful in investigating neurological disorders associated with several regions of the brain, such as spreading depolarizations after traumatic brain injury (*4*, *17, 18*). However, challenges arise when implanting grids with large surface areas, > 10 cm², over the cerebral cortex.

Conventional (research and clinical) ECoG grids are mechanically passive devices, which are surgically laid over the cortex following a craniotomy that exposes the brain (*19*). The size of the craniotomy is at least as large as the footprint of the ECoG grid (in some cases, smaller ECoG strips may be inserted below the skull). Neural recordings are usually performed intraoperatively or over short periods of time (< 30 days) during which the patient remains at the hospital.

A cranioplasty using autologous skull or synthetic materials completes the procedure. The large craniotomies are often a limiting factor in the use of ECoG grids, considering both the risk-benefit ratio and acceptability of the procedure by the patients. Post-surgical complications may arise including inflammatory response and scarring, morphological changes on the brain, and neurological deficiencies (*20*) (*21-23*).

An alternative to planar ECoG grids are stereotactic EEG (electroencephalography) probes: they are implanted through small (< 1 cm²) burr holes in the skull thereby show lower risk profile than ECoG. sEEG is less invasive than Electrocorticography, since electrodes are implanted into the brain without removing the skull, leading to fewer complications, less post-operative pain, and a shorter length of stay in the hospital. On the other hand, investigation of neural activities is limited to small areas, substantially corresponding to the size of the burr holes (< 1 cm²).

The problem at the base of the present invention is that of providing an implantable electrical/electronic biomedical device, such as ECoG grids, that reduce post-surgical complications and could be implanted through minimally invasive surgical procedures, while still keeping a large surface area to be put in contact with a patient's biological surface such as the brain. The present invention addresses at least in part the above problems.

WO2021/005382 discloses a medical device comprising a flexible electrode array having a bend radius of no more than 2mm and a fluidic component configured to change the configuration of the flexible electrode when the fluidic component is fluidically actuated.

### List of references:

1. A. Palmini, The concept of the epileptogenic zone: a modern look at Penfield and Jasper's views on the role of interictal spikes. Epileptic Disorders. 8, 10-15 (2006).
2. J. P. Mullin, D. Sexton, S. Al-Omar, W. Bingaman, J. Gonzalez-Martinez, Outcomes of Subdural Grid Electrode Monitoring in the Stereoelectroencephalography Era. World Neurosurgery. 89, 255- 258 (2016).
3. P. C. De Witt Hamer, S. G. Robles, A. H. Zwinderman, H. Duffau, M. S. Berger, Impact of Intraoperative Stimulation Brain Mapping on Glioma Surgery Outcome: A Meta-Analysis. Journal of Clinical Oncology. 30, 2559-2565 (2012).
4. J. A. Hartings et al., Direct current electrocorticography for clinical neuromonitoring of spreading depolarizations. Journal of Cerebral Blood Flow & Metabolism. 37, 1857-1870 (2016).
5. A. Lecomte, E. Descamps, C. Bergaud, A review on mechanical considerations for chronically- implanted neural probes. 15, 031001 (2018).
6. A. A. Schendel, K. W. Eliceiri, J. C. Williams, Advanced materials for neural surface electrodes. Neuroengineering Materials. 18, 301-307 (2014).
7. D. De Ridder, G. De Mulder, T. Menovsky, S. Sunaert, S. Kovacs, Electrical stimulation of auditory and somatosensory cortices for treatment of tinnitus and pain. Prog Brain Res. 166, 377-388 (2007).
8. M. Lauritzen et al., Clinical Relevance of Cortical Spreading Depression in Neurological Disorders: Migraine, Malignant Stroke, Subarachnoid and Intracranial Hemorrhage, and Traumatic Brain Injury. Journal of Cerebral Blood Flow & Metabolism. 31, 17-35 (2010).
9. R. P. Lesser, N. E. Crone, W. R. S. Webber, Subdural electrodes. Clinical Neurophysiology. 121, 1376-1392 (2010).
10. J. C. Bulacio et al., Long-term seizure outcome after resective surgery in patients evaluated with intracranial electrodes. Epilepsia. 53, 1722-1730 (2012).
11. G. Nune et al., Treatment of drug-resistant epilepsy in patients with periventricular nodular heterotopia using RNS® System: Efficacy and description of chronic electrophysiological recordings. Clinical Neurophysiology. 130, 1196-1207 (2019).
12. M. J. Vansteensel et al., Fully Implanted Brain-Computer Interface in a Locked-In Patient with ALS. New England Journal of Medicine. 375, 2060-2066 (2016).
13. W. Wang et al., An Electrocorticographic Brain Interface in an Individual with Tetraplegia. journalsplosorg. 8, e55344 (2013).
14. J.-J. Mo et al., Motor cortex stimulation: a systematic literature-based analysis of effectiveness and case series experience. BMC Neurology. 19, 48 (2019).
15. J. G. Makin, D. A. Moses, E. F. Chang, Machine translation of cortical activity to text with an encoder-decoder framework. Nature Neuroscience. 23, 575-582 (2020).
16. P. D. Marasco et al., Neurorobotic fusion of prosthetic touch, kinesthesia, and movement in bionic upper limbs promotes intrinsic brain behaviors. Science Robotics. 6, eabf3368 (2021).
17. D. Y. Chung, F. Oka, C. Ayata, Spreading Depolarizations: A Therapeutic Target Against Delayed Cerebral Ischemia After Subarachnoid Hemorrhage. Journal of Clinical Neurophysiology. 33, 196- 202 (2016).
18. J. A. Hartings et al., Spreading depolarisations and outcome after traumatic brain injury: a prospective observational study. The Lancet Neurology. 10, 1058-1064 (2011).
19. T. Kaiju et al., High Spatiotemporal Resolution ECoG Recording of Somatosensory Evoked Potentials with Flexible Micro-Electrode Arrays. Front Neural Circuits. 11, 20 (2017).
20. J. T. Cole et al., Craniotomy: true sham for traumatic brain injury, or a sham of a sham? J Neurotrauma. 28, 359-369 (2011).
21. M. J. Mack, Minimally Invasive and Robotic Surgery. JAMA. 285, 568-572 (2001).
22. S. R. Dashti et al., Operative intracranial infection following craniotomy. FOC. 24, E10 (2008).
23. C. Lin, X. Zhao, H. Sun, Analysis on the risk factors of intracranial infection secondary to traumatic brain injury. Chinese Journal of Traumatology. 18, 81-83 (2015).
24. U.-J. Jeong et al., A minimally invasive flexible electrode array for simultaneous recording of ECoG signals from multiple brain regions. Lab on a Chip. 21, 2383-2397 (2021).
25. M. A. O'Reilly, A. Muller, K. Hynynen, Ultrasound Insertion Loss of Rat Parietal Bone Appears to Be Proportional to Animal Mass at Submegahertz Frequencies. Ultrasound in Medicine & Biology. 37, 1930-1937 (2011).
26. E. M. Lillie, J. E. Urban, S. K. Lynch, A. A. Weaver, J. D. Stitzel, Evaluation of Skull Cortical Thickness Changes With Age and Sex From Computed Tomography Scans. J Bone Miner Res. 31, 299-307 (2016).
27. B. J. Woodington et al., Electronics with shape actuation for minimally invasive spinal cord stimulation. Science Advances. 7, eabg7833 (2021).
28. A. Pal, D. Goswami, R. V. Martinez, Elastic Energy Storage Enables Rapid and Programmable Actuation in Soft Machines. Advanced Functional Materials. 30, 1906603 (2019).
29. J. D. Greer, T. K. Morimoto, A. M. Okamura, E. W. Hawkes, A Soft, Steerable Continuum Robot That Grows via Tip Extension. Soft Robotics. 6, 95-108 (2018).
30. N. D. Naclerio et al., Controlling subterranean forces enables a fast, steerable, burrowing soft robot. Science Robotics. 6, eabe2922 (2021).
31. J. Luong et al., Eversion and Retraction of a Soft Robot Towards the Exploration of Coral Reefs. 2019 2nd IEEE International Conference on Soft Robotics (RoboSoft), 801-807 (2019).
32. E. W. Hawkes, L. H. Blumenschein, J. D. Greer, A. M. Okamura, A soft robot that navigates its environment through growth. Science Robotics. 2, eaan3028 (2017).
33. N. D. Naclerio, C. M. Hubicki, Y. O. Aydin, D. I. Goldman, E. W. Hawkes, Soft Robotic Burrowing Device with Tip-Extension and Granular Fluidization. 2018 IEEE/RSJ International Conference on Intelligent Robots and Systems (IROS), 5918-5923 (2018).
34. I. R. Minev et al., Electronic dura mater for long-term multimodal neural interfaces. Science. 347, 159-163 (2015).
35. N. Vachicouras et al., Microstructured thin-film electrode technology enables proof of concept of scalable, soft auditory brainstem implants. Science Translational Medicine. 11, eaax9487 (2019).
36. G. Schiavone et al., Soft, Implantable Bioelectronic Interfaces for Translational Research. Advanced Materials. 32, 1906512 (2020).
37. F. Fallegger et al., MRI-Compatible and Conformal Electrocorticography Grids for Translational Research. Advanced Science. 8, 2003761 (2021).
38. S. P. Lacour, D. Chan, S. Wagner, T. Li, Z. Suo, Mechanisms of reversible stretchability of thin metal films on elastomeric substrates. Applied Physics Letters. 88, 204103 (2006).
39. I. R. Minev, N. Wenger, G. Courtine, S. P. Lacour, Research Update: Platinum-elastomer mesocomposite as neural electrode coating. APL Materials. 3, 014701 (2015).
40. V. Normand, D. L. Lootens, E. Amici, K. P. Plucknett, P. Aymard, New Insight into Agarose Gel Mechanical Properties. Biomacromolecules. 1, 730-738 (2000).
41. J. Blaber, B. Adair, A. Antoniou, Ncorr: Open-Source 2D Digital Image Correlation Matlab Software. Experimental Mechanics. 55, 1105-1122 (2015).
42. M. M. Swindle, A. Makin, A. J. Herron, F. J. Clubb Jr, K. S. Frazier, Swine as Models in Biomedical Research and Toxicology Testing. Veterinary Pathology. 49, 344-356 (2011).
43. M. Gierthmuehlen et al., Evaluation of µECoG electrode arrays in the minipig: Experimental procedure and neurosurgical approach. Journal of Neuroscience Methods. 202, 77-86 (2011).
44. P. Sauleau, E. Lapouble, D. Val-Laillet, C. H. Malbert, The pig model in brain imaging and neurosurgery. animal. 3, 1138-1151 (2009).
45. Z. Li, H. Yang, G. Wang, X. Han, S. Zhang, Compressive properties and constitutive modeling of different regions of 8-week-old pediatric porcine brain under large strain and wide strain rates. Journal of the Mechanical Behavior of Biomedical Materials. 89, 122-131 (2019).
46. S. Budday, T. C. Ovaert, G. A. Holzapfel, P. Steinmann, E. Kuhl, Fifty Shades of Brain: A Review on the Mechanical Testing and Modeling of Brain Tissue. Archives of Computational Methods in Engineering. 27, 1187-1230 (2020).
47. I. Terem et al., Revealing sub-voxel motions of brain tissue using phase-based amplified MRI (aMRI). Magn Reson Med. 80, 2549-2559 (2018).
48. S. Wurth et al., Long-term usability and bio-integration of polyimide-based intra-neural stimulating electrodes. Biomaterials. 122, 114-129 (2017).
49. A. D. Degenhart et al., Histological evaluation of a chronically-implanted electrocorticographic electrode grid in a non-human primate. 13, 046019 (2016).
50. L. Dejace, H. Chen, I. Furfaro, G. Schiavone, S. P. Lacour, Microscale Liquid Metal Conductors for Stretchable and Transparent Electronics. Adv Mater Technol, 2100690 (2021).
51. Y.-L. Park, B.-R. Chen, R. J. Wood, Design and Fabrication of Soft Artificial Skin Using Embedded Microchannels and Liquid Conductors. IEEE Sensors Journal. 12, 2711-2718 (2012).
52. M. A. Robertson, L. Dejace, S. P. Lacour, J. Paik, Bi-modal control of vacuum-powered soft pneumatic actuators with embedded liquid metal-based strain sensitive skin. 2019 2nd IEEE International Conference on Soft Robotics (RoboSoft), 217-221 (2019).
53. L. Dejace, N. Laubeuf, I. Furfaro, S. P. Lacour, Gallium-Based Thin Films for Wearable Human Motion Sensors. Adv Intell Syst. 1, 1900079 (2019).
54. A. P. Gerratt, H. O. Michaud, S. P. Lacour, Elastomeric Electronic Skin for Prosthetic Tactile Sensation. Advanced Functional Materials. 25, 2287-2295 (2015).
55. F. Fallegger, G. Schiavone, S. P. Lacour, Conformable Hybrid Systems for Implantable Bioelectronic Interfaces. Advanced Materials. 32, 1903904 (2020).
56. J. Viventi et al., Flexible, foldable, actively multiplexed, high-density electrode array for mapping brain activity in vivo. Nature Neuroscience. 14, 1599-1605 (2011).
57. L. Xu et al., 3D multifunctional integumentary membranes for spatiotemporal cardiac measurements and stimulation across the entire epicardium. Nature Communications. 5, 3329 (2014).
58. D.-H. Kim et al., Dissolvable films of silk fibroin for ultrathin conformal bio-integrated electronics. Nature Materials. 9, 511-517 (2010).
59. C. M. Tringides et al., Viscoelastic surface electrode arrays to interface with viscoelastic tissues. Nature Nanotechnology, 1-11 (2021).
60. C. P. Teuwen et al., Quantification of the Arrhythmogenic Effects of Spontaneous Atrial Extrasystole Using High-Resolution Epicardial Mapping. Circulation: Arrhythmia and Electrophysiology. 11, e005745 (2018).
61. A. Farajidavar, Bioelectronics for mapping gut activity. Where the gut meets the brain. 1693, 169- 173 (2018).
62. L. Miller, A. Farajidavar, A. Vegesna, Use of Bioelectronics in the Gastrointestinal Tract. Cold Spring Harbor Perspectives in Medicine. 9 (2019).
63. M. Capogrosso et al., A brain-spine interface alleviating gait deficits after spinal cord injury in primates. Nature. 539, 284-288 (2016).

### SUMMARY OF THE INVENTION

In order to address the limitations of the prior art solutions in the field of implantable devices, in particular to facilitate a minimally invasive approach, the present invention provides for a novel and advantageous design for such devices.

The present invention features an implantable electrical/electronic biomedical device embodied in an implemented example as a deployable ECoG system, assisted with soft robotic actuation, to surgically implant large area and soft microfabricated ECoG grids on the cortex through small surgically obtained cavities (burr holes). The ratio of the ECoG covered cortical area over the burr hole diameter is > 10. The deployment approach relies on a bio-inspired, soft robotic mechanism called eversion (reference 29). The ECoG grid is prepared with the soft e-dura process enabling thin (< 300 µm thick), dura mater-like electrode implants (reference 34).

Generally speaking, the term "eversion" refers to the process of turning inside-out an item. More particularly, in the frame of the present invention, eversion is an actuation mechanism relying on the inside out deployment of an initially inverted thin-walled surface, particularly an electrode structure according to the present disclosure. An "eversion robot" can grow longitudinally, squeeze through small openings and explore constrained environments (references 30-33). Structurally eversion robots are cylindrical sleeves that are folded inwards. When pressurized, the sleeves flip inside out, elongate and move or "grow" forward. The inventors implemented this actuation mechanism to design deployable ECoG strips hosting microelectrodes and built-in strain sensors monitoring in real-time the deployment of the soft implant under the skull. The sensors provide critical feedback on when to stop the eversion actuation. Actuation is controlled through pressurized dispensing of a biocompatible lubricant, e.g. saline solution. Furthermore, the deployable ECoG system is thin and soft and ensures an intimate contact to the curvilinear brain surface. The invention is defined by independent claim 1. Further embodiments are defined in dependent claims 2-16.

Accordingly, one aspect of the invention relates to an implantable electrical/electronic biomedical device comprising:
a) a reservoir defining an inner volume comprising an inlet at a first end and a mechanical support at second, opposed end, and
b) at least one electrode structure deployable by eversion, said electrode structure comprising:
   i) an elastic polymeric support operatively connected to the reservoir's inlet through a first end and to the mechanical support at a second end, and
   ii) at least one conductive track disposed on a surface of said elastic polymeric support.

In particular, before the eversion event is operated,
- said electrode structure is located inside said reservoir inner volume
- said electrode structure comprises a pocket in fluidic connection with the reservoir inner volume and
- said at least one conductive track is not in contact with the reservoir inner volume.

In one embodiment, said at least one conductive track is compliant.

In one embodiment, the device comprises a plurality of electrode structures deployable by eversion. In one embodiment, said at least one conductive track comprises a microelectrode array.

Another non-claimed aspect relates to a method for implanting an implantable electrical/electronic biomedical device according to the invention comprising the step of:
a) deploying at least one electrode structure deployable by eversion by applying a fluidic pressure inside the reservoir.

In one embodiment, said fluidic pressure is applied by a liquid injection.

The at least one electrode structure is intended to be deployed through a hole of a predetermined diameter d and, advantageously, to cover a predetermined length d1 in a direction of deployment dp when deployed. The ratio of the predetermined length d1 and the hole diameter d is greater than ten.

The reservoir includes a fluid and the at least one electrode structure includes an opposite surface S1, opposed to the surface S where the at least one conductive track is arranged, faced to the fluid. The surface on which the conductive track 400 is arranged is not faced to the fluid.

A direction of deployment dp of the electrode structure may be curved or straight. The direction of deployment preferably lays on a plane perpendicular to a direction between the inlet and the mechanical support.

The electrode structure forms part of the reservoir and connects the inlet to the mechanical support 200.

A width of the inlet of the reservoir and a width of the mechanical support in a direction of deployment dp is unchanged by deployment.

In an embodiment, the elastic polymeric support is operatively connected to the reservoir's inlet through the first end of the elastic polymeric support fixed to a connecting structure, wherein the width of the inlet corresponds to a size of an opening of the connecting structure. The opening structure is preferably rigid.

Preferably, the electrode structure includes at least three folding lines L before deployment.

More preferably, the electrode structure includes three or more folding lines L before deployment.

Preferably, the electrode structure includes at least two folding lines L after deployment.

More preferably, the electrode structure includes two folding lines L after deployment.

In one embodiment, a plurality of electrode structures may be arranged in the reservoir to be deployed by eversion through the same hole, such as a burr hole, each one of the electrode structures covering the predetermined length in respective directions of deployment when deployed.

Preferably, each one of the electrode structures is distanced from another one of the electrode structures by a predetermined angular distance.

Further embodiments of the present invention are defined by the appended claims.

The above and other objects, features and advantages of the herein presented subject-matter will become more apparent from a study of the following description with reference to the attached figures showing some preferred aspects of said subject-matter.

### BRIEF DESCRIPTION OF THE DRAWINGS

In the Figures:

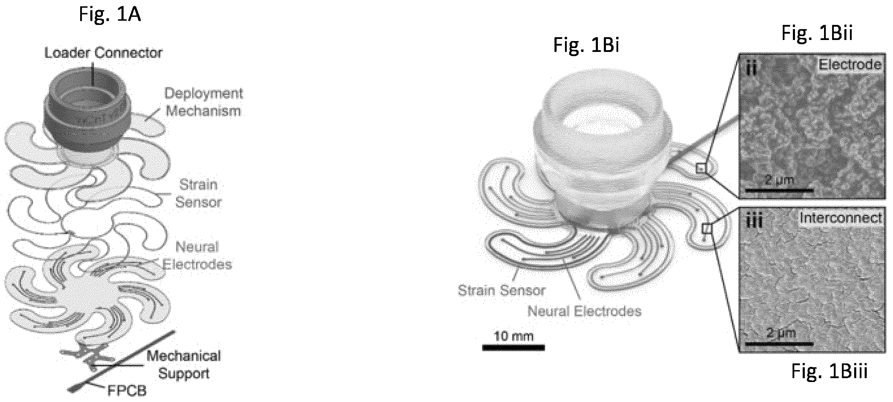

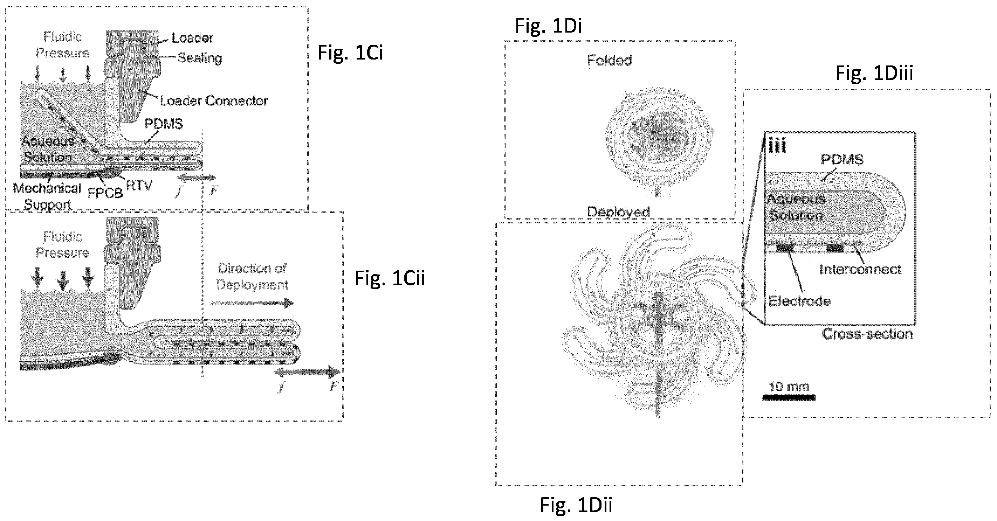

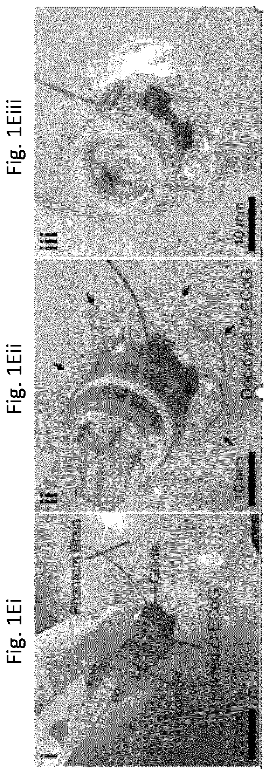

**Fig. 1** **shows the design, fabrication and working principle of *D-*ECoG. (A)** A breakdown image of *D*- ECoG (multi-legged, ML) consisting of everting actuation mechanism, strain sensor and surface electrodes. **(B)** Photographic images of a *D*-ECoG (ML) prototype: (**i**) an overview, (**ii**) a scanning electro-microscope (SEM) image of Pt-PDMS composite and (**iii**) a SEM image of microcracked gold thin-film interconnect. **(C)** Schematic cross-sections of a *D*-ECoG: (**i**) folded and (**ii**) under deployment with a positive fluidic pressure differential. A dashed line indicates the initial position of *D*-ECoG before deployment. *F* and *f* indicate fluidic force and friction, respectively. **(D)** Photographic top views of a *D*-ECoG (ML) prototype: (**i**) folded and (**ii**) fully deployed. (**iii**) A schematic cross-section of a deployed *D*-ECoG showing its air-pocket structure filled with aqueous solution as a lubricant. **(E)** Photographic images of a *D*-ECoG (ML) prototype being deployed in a synthetic brain model: (**i**) positioning a folded *D*-ECoG (ML) in a burr hole (22 mm in diameter) of the synthetic phantom brain consisting of a hydrogel block and a plastic skull, (**ii**) deploying the *D*- ECoG with a positive fluidic pressure differential (arrows indicate deployed electrode arrays) and (**iii**) removing a loader to leave the deployed *D*-ECoG (ML) in position;

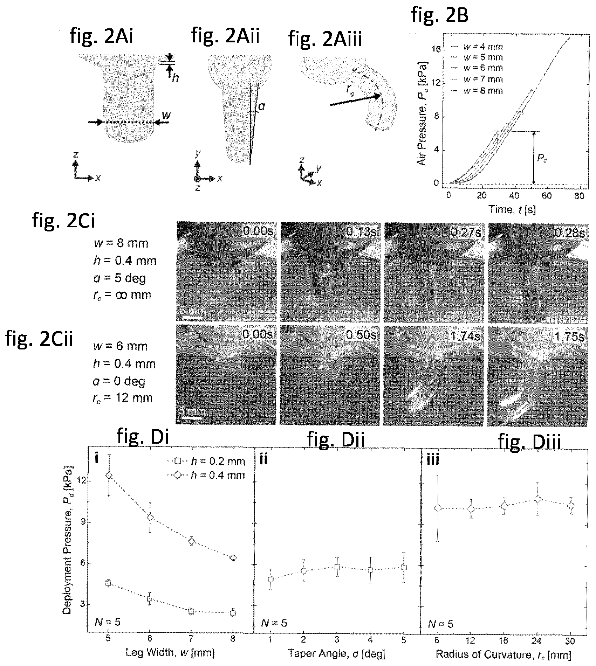

**Fig. 2** **shows deployability of D-ECoG (A)** Definition of design variables: (**i**) leg width *w*, thickness *h* on a straight *D*-ECoG (single-legged, SL), (**ii**) taper angle *a* on a tapered *D*-ECoG (SL), and (**iii**) radius of curvature *r_{C}* on a curved *D*-ECoG (SL). **(B)** Profiles of air pressure *Pₐ* inside straight *D-*ECoG (SL) with respect to time *t,* depending on different width *w*. By default, *h = 0.4* mm, *a* = 0 deg, and *r_{C}* = ∞ mm. **(C)** Sequential images of deployment of *D*-ECoG strips, (**i**) a tapered *D*-ECoG (SL) and (**ii**) a curved *D*-ECoG (SL). *w*, *h, a*, and *r_{C}* are described in the image. **(D)** Deployment pressure *Pd* depending on various design variables: (**i**) leg width *w* and thickness *h*, (**ii**) taper angle *a*, and (**iii**) radius of curvature *r_{C}*. By default, *h* = 0.4 mm, *w* = 6 mm, *a* = 0 deg, and *r_{C}* = ∞ mm. Each data point in **D** is an average of five different measurements (*N* = 5) with a single specimen. Error bars indicate ±1 standard deviation;

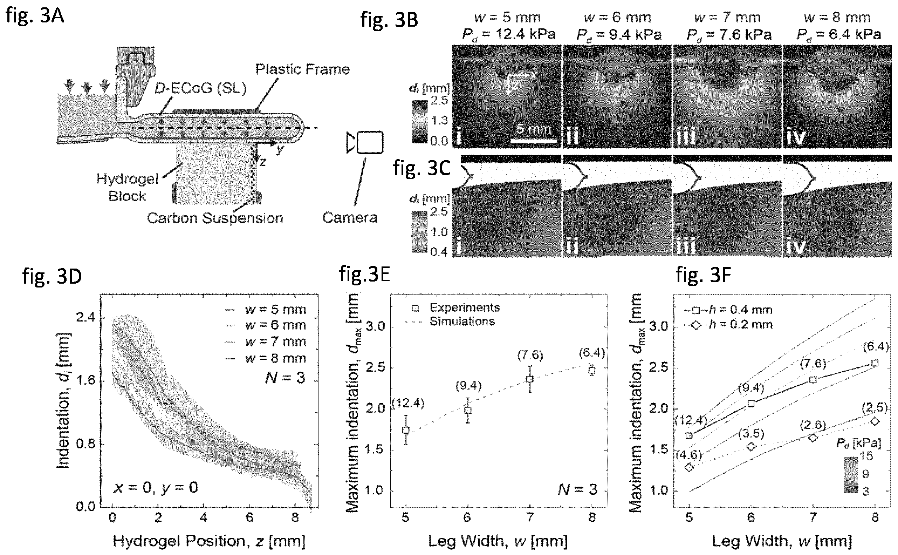

**Fig. 3** **shows Indentation of a phantom brain depending on various *D*-ECoG shapes (A)** A schematic cross-section of a straight *D*-ECoG (SL) (*h* = 0.4 mm and *a* = 0 deg) with experimental set-up. The origin of hydrogel position *z* and indentation *dᵢ* is on the boundary between the lowest bottom of *D*-ECoG and the hydrogel block. **(B)** Color maps of *dᵢ* of the hydrogel block indented by *D*-ECoG with different *w*: (**i**) *w* = 5 mm, (**ii**) *w* = 6 mm, (**iii**) *w* = 7 mm and (**iv**) *w* = 8 mm. Each *D*-ECoG design is inflated with its corresponding *Pd* shown in **Fig. 2D-i****. (C)** Numerical estimation on *dᵢ* of the hydrogel block indented by *D*-ECoG with different *w*: (**i**) *w* = 5 mm, (**ii**) *w* = 6 mm, (**iii**) *w* = 7 mm and (**iv**) *w*= 8 mm. Each *D*-ECoG design is inflated with its corresponding *Pd* shown in **Fig. 2D-i****. (D)** Profiles of *dᵢ* with respect to z depending on different *w*. Each solid line is an average of three measurements (*N* = 3) using the same *D*-ECoG sample. Shaded areas show a range of the minimum and the maximum *dᵢ*. **(E)** Maximum indentation *d*ₘₐₓ of various *D*-ECoG with different *w* when inflated with its corresponding *Pd.* Each data point is an average of three different measurements (*N* = 3) using the same *D*-ECoG sample. Error bars indicate ±1 standard deviation. Dashed line presents calculations of the FEM model. **(F)** Numerical estimation on *d*ₘₐₓ of various *D*-ECoG with different *w*, *h,* and *P_{d}.* The dashed line and colored solid lines are *d*ₘₐₓ for the *D*-ECoG with *h* = 0.4 mm and *h* = 0.2 mm, respectively. Numbers in the brackets in **E** and **F** are corresponding *Pd* for the given *w* and *h*;

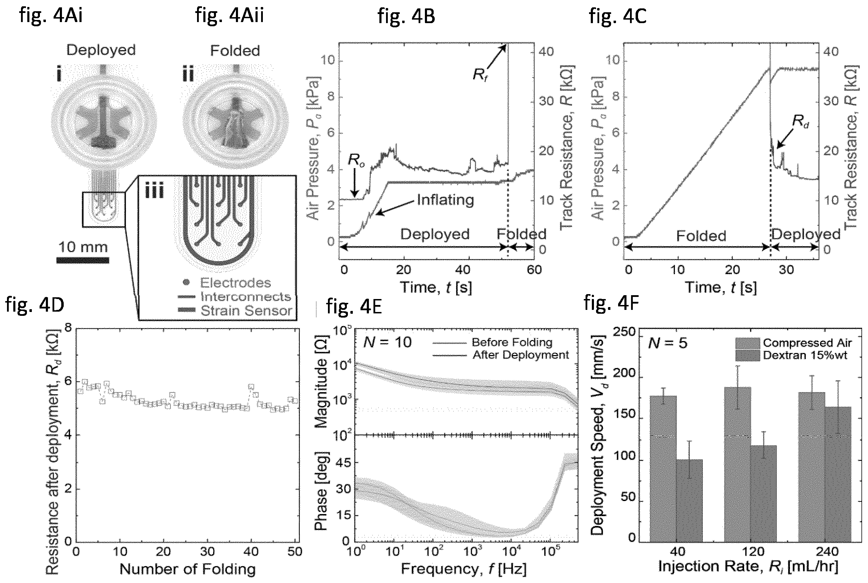

**Fig. 4** **shows verification of functionalities of *D*-ECoG (A)** Photographic top views of a functional *D*-ECoG (SL) prototype: (**i**) deployed and (**ii**) folded. (**iii**) A layout of electrodes, interconnects, and strain sensor. **(B)** Profiles of internal air pressure *Pₐ* and track resistance *R* of strain sensor with respect to time *t* during folding and **(C)** deployment. *Rₒ, R_{f},* and *R_{d}* indicate the initial track resistance before folding, track resistance after folding, and track resistance after deployment, respectively. **(D)** Track resistance after deployment *R_{d}* with respect to the number of folding. **(E)** Electrical impedance magnitude (top) and phase (bottom) of 10 channels (*N* = 10) on the functional *D*-ECoG (SL) before folding (blue) and after deployment (red) as a function of frequency. **(F)** Deployment speed *V_{d}* of *D*- ECoG (SL) filled with different fluids as a function of syringe pump's injection rate *Rᵢ*. Each data point is an average of five different measurements (*N* = 5) using the same *D*-ECoG sample. Error bars indicate ±1 standard deviation;

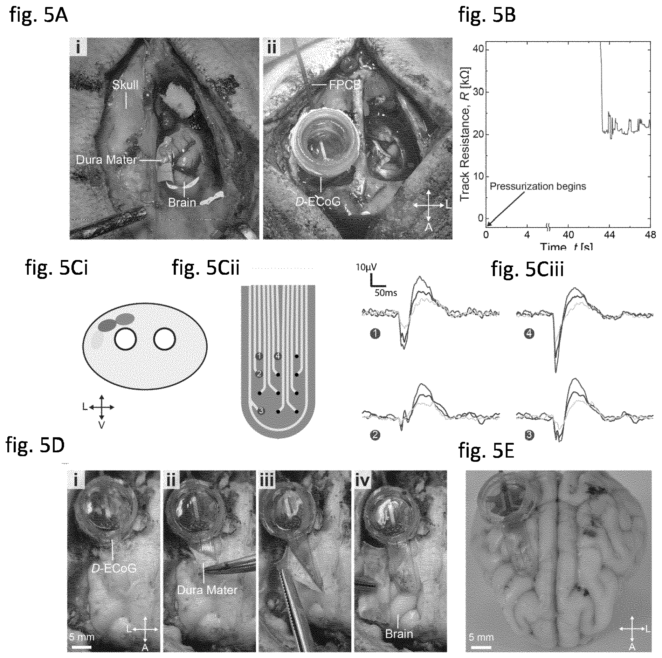

**Fig. 5** **shows an *In-vivo* proof-of-concept of the deployment of the D-ECoG at the surface of the brain in a minipig model and recording somatosensory evoked potentials (SSEPs) from snout stimulation.** (A) Surgical access to deploy the soft implant on the brain: (i) overview of the access to the brain, (ii) *D*-ECoG system after placement on the brain. (B) An electrical track resistance change of the strain sensor during the deployment procedure. (C) Recording of the SSEPs on the brain after deployment of the *D*-ECoG: (i) location of the 3 placements of stimulation on the snout, (ii) electrode design (iii) SSEPs from the snout stimulation according to the snout stimulation position (color code). (D) Extraction of the *D*-ECoG after the brain perfusion showing the deployed soft array. Images are arranged in the chronological order from i to iv. (E) Photograph of the extracted brain with the position of the electrode array depicted schematically on the contra-lateral hemisphere;

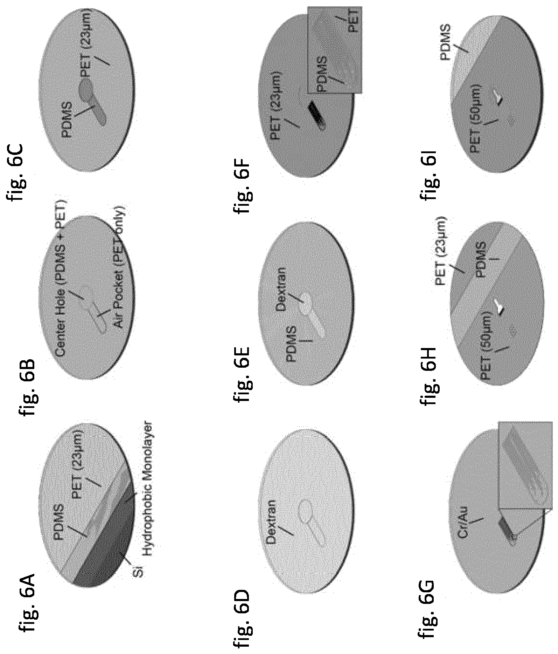

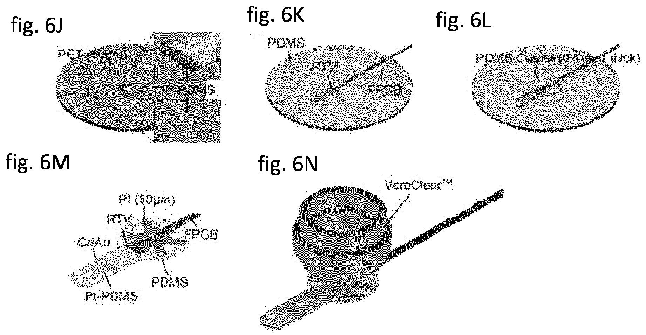

**Fig. 6** **shows a fabrication process of *D*-ECoG (A)** Preparation of the PDMS Top layer (deposition of a hydrophobic monolayer, blade-coating of a 200-µm-thick PDMS layer, and lamination of a 23-µm-thick PET film), **(B)** cutting the entire PDMS Top layer through with center hole pattern while selectively cutting the 23-µm-thick PET film with air pocket pattern, **(C)** delamination of the 23-µm- thick PET film patterned as the air pocket and the center hole, followed by O₂ plasma activation, **(D)** spin coating of a Dextran layer, **(E)** removing of the Dextran layer on the 23-µm-thick PET mask and blade-coating of a 100-µm-thick PDMS Bottom layer, **(F)** lamination and laser cutting of a 23-µm- thick PET film with interconnects and strain sensor patterns as a negative mask, **(G)** thermal evaporation of chromium (5-nm-thick) and gold (35-nm-thick), **(H)** fabrication of an encapsulating PDMS layer by laminating 23-µm-thick and 50-µm-thick PET films on both side of a 100-µm-thick PDMS layer, **(I)** bonding of the encapsulating PDMS layer onto the PDMS Bottom layer using O₂ plasma activation, **(J)** coating of platinum (Pt)-PDMS composite onto electrode sites and comb-like patterns for FPCB connection, **(K)** assembling of a FPCB and passivation of the connecting cite with RTV, **(L)** cutting the entire assembled PDMS layer through with the *D*-ECoGs pattern, **(M)** removing of the Dextran sacrificial layer inside the *D*-ECoGs with water and assembling of a 50-µm-thick polyimide (PI)-made mechanical support, **(N)** assembling of a 3D-printed loader connecter and center cylinder that is separately prepared with injection molding of PDMS;

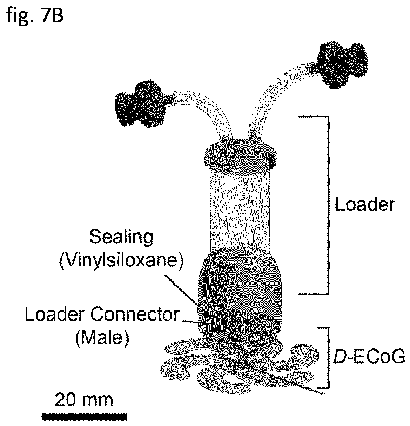

**Fig. 7** **shows a structure and assembly of *D*-ECoG loading system (A)** A 3D image of the loader that consists of female loader connector, loader chamber, and chamber cover all 3D-printed with VeroClear^{™} (Stratasys, Ltd.) connected to silicone tubing (1.6 mm in inner diameter) and luer lock male tubing connectors. **(B)** A 3D image of the fully assembled *D*-ECoG loading system in which the *D*-ECoG is temporarily bonded with the loader using Vinylsiloxane elastomer (Flexitime^{®} Correct Flow, Kulzer GmbH);

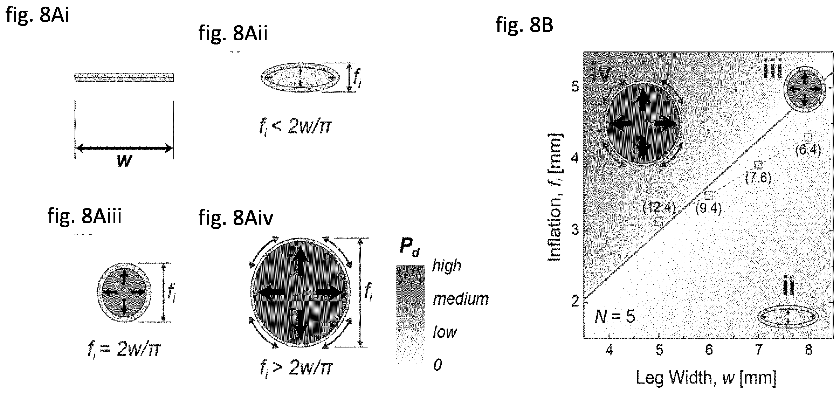

**Fig. 8** **depicts the inflation of *D*-ECoG strips with different leg widths (A)** Schematic cross-sections of a *D*-ECoG strip with leg width *w* depending on deployment pressure *P_{d}.* (**i**) When depressurized (*P_{d}* = 0), the *D*-ECoG strip is flat on 2D plain without inflation distance (*fᵢ =* 0). (**ii**) When *Pd* is not high enough to deform the *D*-ECoG strip into a perfect circular cross-section, the air pocket has an oval cross- section whose *fᵢ < 2w*/*π* without any circumferential stretch on skin. (**iii**) When *Pd* is just enough to have the *D*-ECoG inflated to a perfect circular cross-section, *fᵢ* can be given as *fᵢ = 2w*/*π.* The *D*-ECoG skin still does not experience any circumferential stretch. (**iv**) When Pd is higher than that in the case shown in **iii,** the *D*-ECoG skin undergoes a circumferential stretch that allows the circular cross- section radially expands, resulting in having its *fi* increased *fᵢ > 2w*/*π.* **(B)** Averaged *fᵢ* of 0.4-mm-thick straight, single-legged *D-*ECoG strips with different w ranging from *w* = 5 mm to *w* = 8 mm (red squares) at their corresponding *Pd* (parentheses). Schematic cross-sectional images in the figure correspond to the cases in **A-i, A-ii** and **A-iii.** Each data point is an average of five different measurements (*N* = 5) using the same *D*-ECoG sample. Error bars indicate ±1 standard deviation. A solid blue line indicates theoretical inflation when a *D*-ECoG strip deforms into a perfect circular cross-section without a circumferential stretch on its skin as discussed in the case **A-ii.** The *fᵢ* of *D*- ECoG strips marked as red squares show that all *fi* are either on the sold blue line (*w* = 5 and 6 mm, the case of **A-ii**) or under the solid line (*w* = 7 and 8 mm, the case of **A-i**), meaning that there is no visible stretch on the *D*-ECoG's skin in circumferential direction during deployment. *D*-ECoG strips with thickness thinner than *h* = 0.4 mm will also have no stretch on their elastomeric skin, as their *Pd* will be even lower than those of the 0.4-mm-thick *D*-ECoG samples, implying that their *fᵢ* will be all lower than the red squares with the red dashed line. Therefore, it is possible to conclude that elongation of the *D-*ECoG skin will be negligible within a design parameter space of 5 mm < *w* < 8 mm and *h* < 0.4 mm;

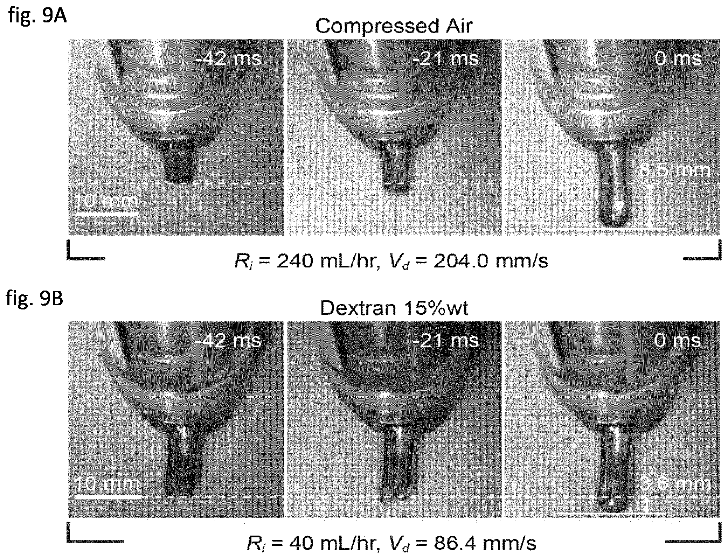

**Fig. 9** **shows sequential images of deployment of a straight D-ECoG (SL) with different fluid media**
   (**A**) Deployment using a loader filled with a mix of 15wt% Dextran solution and compressed air at *Rᵢ* = 240 mL/hr (**B**) Deployment using a loader only filled with 15wt% Dextran solution at *Rᵢ* = 40 mL/hr. Dashed lines indicate position of the tip edge of the *D*-ECoG at 42 ms before the full deployment;
**Fig. 10** **depicts an embodiment of an experimental set-up of a system according to the invention** A schematic image of experimental set-up for in-vitro characterization of folding and deployment of a *D*-ECoG strip;
**Fig. 11** **shows a stress-strain curves for PDMS and hydrogel (A)** A normal stress measurement with respect to engineering strain of the medical-grade PDMS used in this work (LSR M130, Elkem Silicones). (**B**) A normal stress measurement with respect to engineering strain of the Agarose hydrogel block used in this work (Agarose DNA Grade (100 bp - 23 kb), Electran for electrophoresis);
**Fig. 12** **depicts an embodiment of an experimental set-up of a system according to the invention** A schematic image of an experimental set-up for electrochemical impedance spectroscopy (EIS) in 3-electrode configuration;
**Fig. 13** **depicts a schematic configuration of a device of the invention according to one embodiment in a rest state;**
**Fig. 14** **depicts a schematic configuration of a device of the invention according to one embodiment in a semi-deployed state;**
**Fig. 15** **depicts a schematic of the eversion process of the device of** **fig. 14** at an initial phase of eversion;

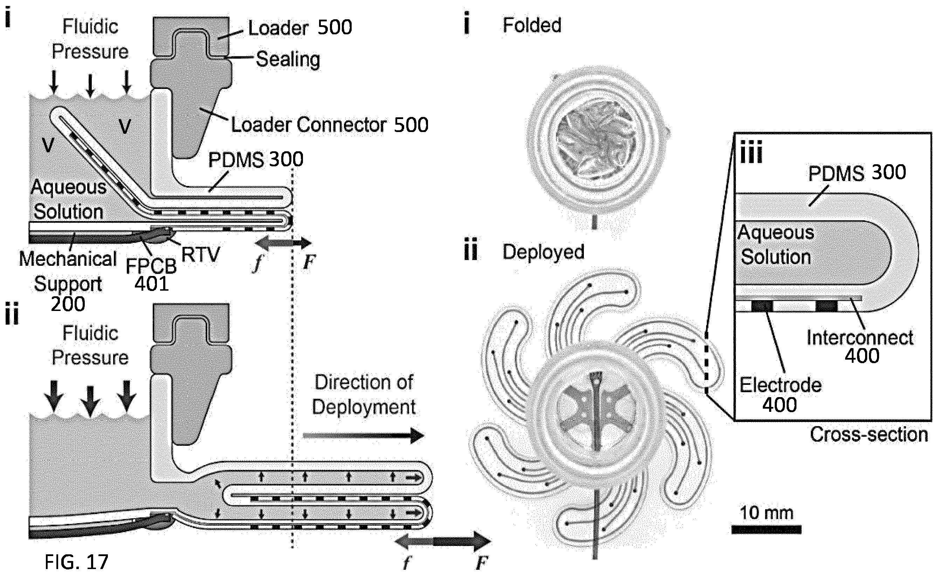

**Fig. 16** **depicts a schematic of the eversion process of the device of** **figure 14****,** at full deployment of the device upon total eversion; **Fig. 17** **shows the eversion process of a device of the invention according to a second embodiment;** the device comprises six electrode structures in a tentacle configuration.

### DETAILED DESCRIPTION OF THE INVENTION

The subject-matter described in the following will be clarified by means of a description of those aspects which are depicted in the drawings. It is however to be understood that the scope of protection of the invention is not limited to those aspects described in the following and depicted in the drawings; to the contrary, the scope of protection of the invention is defined by the claims. Moreover, it is to be understood that the specific conditions or parameters described and/or shown in the following are not limiting of the scope of protection of the invention, and that the terminology used herein is for the purpose of describing particular aspects by way of example only and is not intended to be limiting.

Unless otherwise defined, technical and scientific terms used herein have the same meaning as commonly understood by one of ordinary skill in the art to which this invention belongs. Further, unless otherwise required by the context, singular terms shall include pluralities and plural terms shall include the singular. The methods and techniques of the present disclosure are generally performed according to conventional methods well known in the art and as described in various general and more specific references that are cited and discussed throughout the present specification unless otherwise indicated. Further, for the sake of clarity, the use of the term "about" is herein intended to encompass a variation of +/- 10% of a given value.

Non-limiting aspects of the subject-matter of the present disclosure will be described by way of example with reference to the accompanying figures, which are schematic and are not intended to be drawn to scale. For purposes of clarity, not every component is labelled in every figure, nor is every component of each aspect of the invention shown where illustration is not necessary to allow those of ordinary skill in the art to understand the invention.

The following description will be better understood by means of the following definitions.

As used in the following and in the appended claims, the singular forms "a", "an" and "the" include plural referents unless the context clearly dictates otherwise. Also, the use of "or" means "and/or" unless stated otherwise. Similarly, "comprise", "comprises", "comprising", "include", "includes" and "including" are interchangeable and not intended to be limiting. It is to be further understood that where for the description of various embodiments use is made of the term "comprising", those skilled in the art will understand that in some specific instances, an embodiment can be alternatively described using language "consisting essentially of" or "consisting of."

In the frame of the present disclosure, the expression "operatively connected" and similar reflects a functional relationship between the several components of the device or a system among them, that is, the term means that the components are correlated in a way to perform a designated function. The "designated function" can change depending on the different components involved in the connection. Likewise, any two components capable of being associated can also be viewed as being "operably couplable," to each other to achieve the desired functionality. A person skilled in the art would easily understand and figure out what are the designated functions of each and every component of the device or the system of the invention, as well as their correlations, on the basis of the present disclosure.

Some of the materials used in accordance with the present invention for the electrode structure are soft polymers. In the frame of the present disclosure, a "soft" material is any material that is either compressible, reversibly compressible, elastic, stretchable or any combination thereof. Whenever present, the term "stretchable" refers to the elastic behavior of an item and is herein used to mean an intrinsic or engineered property of a material or structure that allows such material or structure to withstand a large elongation or multidirectional strain upon a strain stress, upon a single or multiple cycles, comprised between 1 and 500%, typically of >5% of the elongation of a soft structure at rest, such as for instance more than about 10%, more than about 20%, more than about 50%, more than about 100% or even more than about 200% of a soft structure at rest without cracking or loss of its physical and/or mechanical properties, which represents an advantage in those contexts and/or body structures in which several cycles of mechanical stresses over time can be foreseen. According to the present invention, refers is made to "an elastic polymeric support" to indicate a support substantially or entirely made of a soft polymeric material, typically a stretchable polymeric material according to the above definitions.

A "compliant electrode" is any structure or element able to deliver an electric current, and adapted to change its shape according to the shape change of the support it adheres to without substantially compromising mechanical or electrical performance. Examples of complaint electrodes known in the art include metal thin-films (including patterned electrodes, of out-of-plane buckled electrodes, and corrugated membranes), metal-polymer nano-composites, carbon powder, carbon grease, conductive rubbers or paints, a review of which is provided by Rosset and Shea (Applied Physics A, February 2013, Volume 110, Issue 2, pp 281-307).

In one embodiment, stretchable electrodes as the one described in International Patent Application WO 2004/095536 can be used. Alternatively or additionally, tubular or plain elements filled with a ionic liquid, a hydrogel or with liquid metals such as mercury or gallium, or even alloys, oxides or combinations thereof, can be used.

The term "flexible" is used herein to refer to elements of the device of the invention that can perform a deflection. Generally speaking, the term "deflection" refers to any displacement, expansion, contraction, bending, torsion, twist, linear or area strain, or any other kind of deformation, of at least a portion of the polymeric structure.

The expressions "film", "membrane" or "thin film" relate to the thin form factor of an element of the device of the invention such as conductive tracks or polymeric substrates. Generally speaking, a "film", "membrane" or "thin film" as used herein relates to a layer of a material having a thickness much smaller than the other dimensions, e.g. at least one fifth compared to the other dimensions.

Typically, a film is a solid layer having an upper surface and a bottom surface, with any suitable shape, and a thickness generally in the order of micrometers or millimetres, depending on the needs and circumstances, e.g. the manufacturing steps used to produce it. In some embodiments, films according to the invention have a thickness comprised between 0.1 µm and 5 mm, such as between 5 µm and 5 mm, between 5 µm and 1 mm, between 10 µm and 1 mm, between 5 µm and 500 µm, between 50 µm and 500 µm between, between 50 µm and 150 µm, 100 µm and 500 µm or between 200 µm and 500 µm.

The expression "conductive track" refers to any film, path, stripe, strand, wire or the like which is electrically conductive in nature. For the sake of clarity, the word "electrode" is herein used to mean the distal part of a conductive track which is in direct contact with a surface, such as a subject's tissue, and/or to pads of a circuit board. Conductive tracks according to the present disclosure are used to connect and/or close an electrical circuit, and are thus usually electrical connectors or "interconnects". A conductive track is generally a metallic element that conducts an electric current toward or away from an electric circuit, but can be made of any suitable electrically conductive material, including but not limited to metals such as Au, Pt, Al, Cu and the like, as well as any alloy, oxides and/or combinations thereof; conductive polymeric materials; composite material such as polymeric materials embedding metal particles and/or metal strands or stripes, including insulating materials functionalized with electrically conductive flakes or fibers, for example carbon-filled polymers; liquid metals, including alloys or oxides thereof, such as gallium; electrically conductive inks; as well as any suitable combination thereof.

The electrode material can be deposited using a variety of techniques known in the art including, but not limited to, printing, pad printing, screen printing, silk screening, flexography, gravure, offset lithography, inkjet, painting, spraying, soldering, bonding deposited using touch-less technologies or otherwise transferred onto the surface of the membrane. In an embodiment, the electrode can be formed by depositing an electrically conductive coating or layer by spraying a preselected onto the designated surface region. Alternatively, the electrode can be formed by depositing the electrically-conductive material onto a region of the membrane by vacuum deposition or printing the electrically conductive material on the designated surface region. This provides an electrically conductive coating of a desired thickness and a relatively uniform electrode through the desired area. Printing processes can include pad printing, screen printing and the like. Touch-free technologies such as positive material deposition of ink such as from a syringe or similar device can also be used to transfer conductive film or ink onto the membrane or substrates that are sensitive to pressure.

The term "subject" as used herein refers to mammals. For example, mammals contemplated by the present invention include humans, primates, domesticated animals such as cattle, sheep, pigs, horses, laboratory rodents and the like.

As used herein, the terms "operator" and "user" may refer to a medical practitioner, surgeon, imaging technician, or other individual or group of individuals involved in operating medical instruments, devices and equipment during a medical procedure.

For "cavity" or "recess" is herein meant any natural or artificially created (e.g. surgically) path in a subject's body that allows for reaching a biological surface inside said subject's body, preferably in a minimally invasive manner in the case of artificially created cavities. Accordingly, the definition is meant to include bodily cavities such as the cranial cavity, the spinal cavity, the ventral body cavity, the thoracic cavity, the abdominopelvic cavity, the abdominal cavity or the pelvic cavity, as well as a brain sulcus, a lumen, a sinus, a ventricle and the like.

Within the meaning of the present invention, a "fixed implant" defines a biomedical device having the ability to conform to established and/or customised surgical procedures and to reside in vivo without producing adverse biological reactions over extended periods of time, such as for instance over 7 days. Still within the meaning of the present invention, a "removable implant" defines a biomedical device having the ability to conform to established and/or customised surgical procedures and to reside in vivo for a limited amount of time, such as for instance the time of a surgical operation.

The term "diagnosing" as used herein refers to assessing whether a subject suffers from a disease or not. The term includes individual diagnosis of the disease, or its symptoms, as well as continuous monitoring of a patient. Monitoring, i.e. diagnosing the presence or absence of a disease or the symptoms accompanying it at various time points, includes monitoring of subjects known to suffer from a disease as well as monitoring of subjects known to be at risk of developing a disease. Furthermore, monitoring can also be used to determine whether a subject is treated successfully or whether at least symptoms of a disease can be ameliorated over time by a certain therapy. Diagnosing as used herein also refers to diagnosing a predisposition of a disease and, thus, predicting whether a subject is at increased risk of developing a disease within a predictive window starting from the time when the sample to be analyzed has been taken. Preferably, the predictive window is at least three months, six months, one year, two years, five years, ten years or up to the entire life span of the subject. A subject is at increased risk if the probability by which it will develop the disease is statistically significantly increased with respect to the average or mean probability, i.e. the prevalence for the disease in the respective population from which the analysed subject originates.

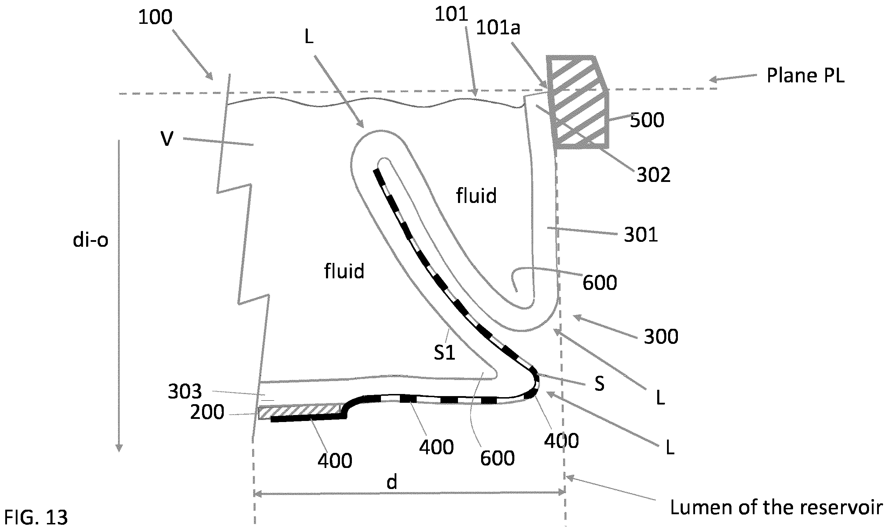

With reference to **Figs. 13 to 16** depicting one embodiment of the invention, an implantable electrical/electronic biomedical device is shown comprising:
a) a reservoir 100 defining an inner volume V comprising an inlet 101 at a first end 302 and a mechanical support 200 at a second, opposed end 303, and
b) at least one electrode structure 300 deployable by eversion, said electrode structure 300 comprising:
   i) an elastic polymeric support 301 operatively connected to the reservoir's inlet 101 through a first end and to the mechanical support 200 at a second end, and
   ii) at least one conductive track 400 disposed on a surface of said elastic polymeric support 301.

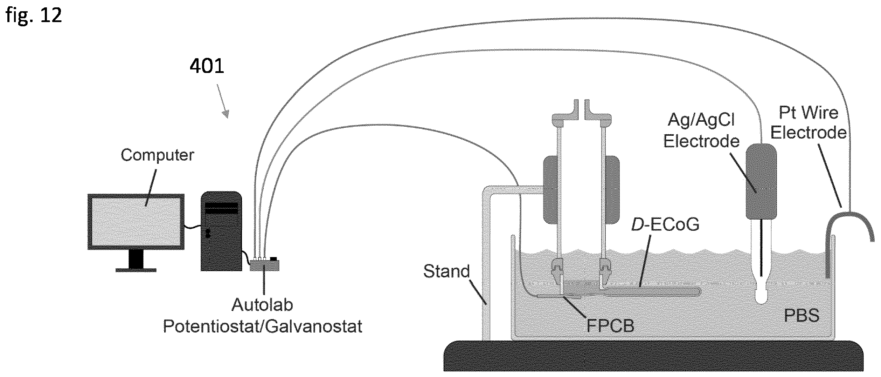

As shown in **Figs. 13 to 16****,** the at least one conductive track 400 is configured to be operatively connected to an external or integrated device 401 (as schematically represented in **fig. 12**), such as for instance a printed circuit board (PCB), including flexible PCBs (FPCB), a power supply and the like. As a way of example, a thin flexible printed circuit board 401 may be interfaced with the interconnects 400 through electric pads to form a low geometrical profile connector.

Additionally, in embodiments of the invention, a connector structure 500 may be present. The connector structure 500 is located at the level of the inlet 101 at the first end of the reservoir 100, and is designed to connect the device, and the reservoir 100 in particular, to additional elements such as tubes or further reservoirs, to be used as loader chambers to inject a fluid into the reservoir 100 via the inlet 101 for deployment by eversion of the electrode structure 300. The loader chamber may, in turn, comprise a loader connector configured to be coupled to the connector structure 500 of the device. As a way of example, the connector structure 500 may comprise a threading for screwing the device of the invention with external structures such as loaders.

The elastic behavior of the support 301 is provided by the materials it is substantially composed of. In this context, in preferred embodiments the support substrate 301 is substantially made of a soft polymeric material, or combinations of many soft polymeric materials, particularly biocompatible ones, or polymeric materials coated with soft polymeric material or hydrogels, or made of composite materials. Preferred soft materials are elastomeric materials, thermoplastic elastomers (a class of copolymers or a physical mix of polymers, usually a plastic and a rubber, which consist of materials with both thermoplastic and elastomeric properties), foams, gels or hydrogels. As a way of example, the substrate 301 can be substantially composed of one or more polymer selected from a non-exhaustive and non-limiting list comprising thermoset materials such as alkyds, epoxies, polyester thermosets, unsaturated polyesters, polyurethane, bis-maleimides (BMI), silicone materials such as polydimethylsiloxane (PDMS) or other medical grade silicones and any combination thereof; nitrile rubber, latex, hydrogels such as polyhydroxymethylacrylate and its derivatives, poly(lactic-co-glycolic acid), lactide and glycolide polymers, caprolactone polymers, polyisoprene (synthetic rubber), polyurethane foam (foam rubber), XPS foam, styrenic block copolymers, elastomeric alloys, hydroxybutyric acid, polyanhydrides, polyesters, polyphosphazenes, polyphosphoesters and poly(glycerol sebacate acrylate), polypropylene, polypropylenoxide or their derivatives, polymethylenoxide or its derivatives, polyethylene or its derivatives such as polyethylene glycole (PEG), polyethylenoxide or their derivatives, polyacrylate or its derivatives, poly(vinyl alcohol) (PVA), poly(lactic acid), poly(methacrylic acid), and copolymers, poly(vinylpyrrolidone) (PVP) and combinations thereof; as well as any combination of the foregoing. These materials can be mixed with particle such as carbon nanotube, gold particles, platinum particles or diamond particle or any combinations such as to improve mechanical, electrical or thermal properties.

Still preferably, the support 301 is reversibly stretchable (elastic). In particular, support 301 can withstand an elongation or multidirectional strain, upon a single or multiple cycles, comprised between 1 and 500%, preferably at least 5%, such as about 50%, about 100% or about 200%, of its size at rest without cracking or loss of its mechanical properties. Further, the support 301 has a Young's modulus comprised between about 1 kPa and 1 GPa, such as for instance between about 100 kPa to about 1 GPa, between about 100 kPa to about 1 GPa, between about 5 MPa to about 1 GPa, between about 100 kPa to about 100 MPa, between about 100 kPa to about 5 MPa, between about 10 kPa to about 300 kPa or between about 10 kPa to about 10 MPa, preferably between about 1 MPa to about 10 MPa, which are suitable ranges of values matching the Young's modulus of many biological tissues and surfaces to avoid mechanical mismatches between said tissues and a biomedical device, and/or for mimicking physical and/or mechanical properties of bodily tissues. In the frame of the present invention, "physical and/or mechanical properties" means, by way of examples, stress-strain behaviour, elastic modulus, fracture strain, conformability to curvilinear surfaces, thickness, area and shape which have to be as similar as possible to those to be found in tissues of a subject's body.

In a most preferred embodiment, the support 301 is an elastomeric substrate, for instance a PDMS substrate. The support 301 is preferably implemented as a thin film substrate having a flat appearance; a PDMS substrate having a thin film thickness and form factor presents several advantages in the frame of medical devices according to the invention, such as biocompatibility, mechanical compliance with regards to body tissue, possibility to be sterilized and high stretchability. All these features make of a PDMS thin film a particularly advantageous choice in the context of an implantable biomedical device.

The support 301 is preferably anchored to the mechanical support 200; the main aim of the mechanical support 200 stands in the avoidance of the system from inflating and compressing a subject's biological surface, like the brain, by bulging out with the positive pressure differential upon the deployment of the at least on electrode structure 300 via application of a fluidic pressure therein.

For the sake of clarity, it is convenient to specify that the electrical/electronic biomedical device comprises at least two "states" or configurations, namely a so-called "rest" state and a "fully deployed" state (**Figs**. **13 to 15**). Intermediate states are envisageable albeit possibly not functionally useful to correctly implant and operate the device.

In the rest state, the device is not deployed, the eversion event did not take place.

The electrode structure 300 connects the inlet 101 with the mechanical support 200. The inlet 101 includes a peripheral portion 101a. The peripheral portion 101 may be structurally formed by the connector structure 500, in particular by an inner surface of the connector structure 500, for instance an inner circumferential surface. A projection of the peripheral portion 101 in the direction di-o (**fig. 13**) between the inlet 101 and the mechanical support 200 defines a lumen of the reservoir 100. The direction di-o is an axial direction, i.e. a direction perpendicular to a plane PL passing through the inlet 101. In use, the lumen falls inside the burr hole having a diameter d (**fig. 13**). At rest, i.e. before deployment, the electrode structure 300 may be located inside the lumen (**fig. 13**). The electrode structure 300 forms part of the reservoir 100. The electrode structure 300 forms a lateral side of the reservoir 100, between the inlet 101 and the mechanical support 200. The electrode structure 300 is folded at rest inside the lumen. The volume V as well is inside the lumen, at rest. The volume V is also indicated as the inner volume V inside the reservoir 100, meaning the volume V which is separated by the electrode structure 300 with respect to an outer volume which is outside the reservoir 100. The electrode structure 300 is folded several times. For instance, in the example of **fig. 13****,** the electrode structure 300 includes three folding lines 3. A folding line is a line L along which two portions of a surface S (of the electrode structure 301 where the conductive tracks 400 are arranged) are reciprocated or a line L along which two portions of an opposite surface S1 (opposed to the surface S) are reciprocated. Preferably, along a folding line L, the two portions are reciprocated to form an acute angle, such as less than 60°, preferably less than 30°, more preferably less than 10 degrees. Two portions reciprocated along one folding line L may form an angle different from the angle formed by two other portions along a different folding line L. In the rest state, the device is in a collapsed configuration that facilitates manipulation thereof and implantation in a subject.

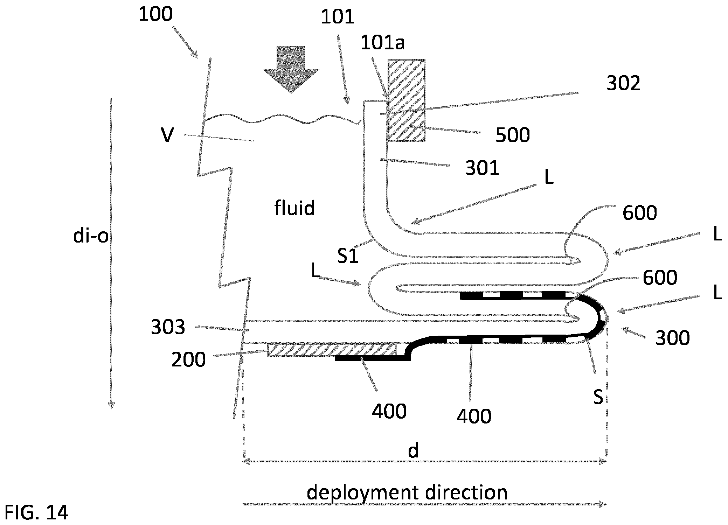

In **Fig. 14** a possible, non-limiting and schematic configuration of the device in a rest state is shown. In this embodiment, at rest, at least one portion of the at least one electrode structure 300 is outside the lumen of the inlet 101. Such at least one portion, however, in use at rest, is inside the lumen of the burr hole of diameter d (**fig. 14**). The at least one electrode 301 is folded along folding lines L similarly to what already described above. The at least one electrode 301 forms a loop that delimits a main chamber and two secondary chambers 600 in the volume V. The loop may have a convoluted and enwrapped configuration as well. The secondary chambers 600 may be completely separated between them, by means of reciprocating portions of the opposite surface S1 touching one with the other, or may be pockets inside the reservoir 100 not interrupting the fluid communication within the volume V, as shown in **Figs. 13** **and** **14****.** The electrode structure loop is configured with the opposite surface S1 of the elastic polymeric support 301 faced with the fluid in the volume V and to isolate the conductive tracks 400, which rest unfaced to the fluid (in the outer volume). At the same time, a second, opposed surface comprising the conductive tracks 400 forms the inner portion of the loop, thereby protecting the tracks 400 from the external environment before the eversion event, and from the content of the reservoir as well.

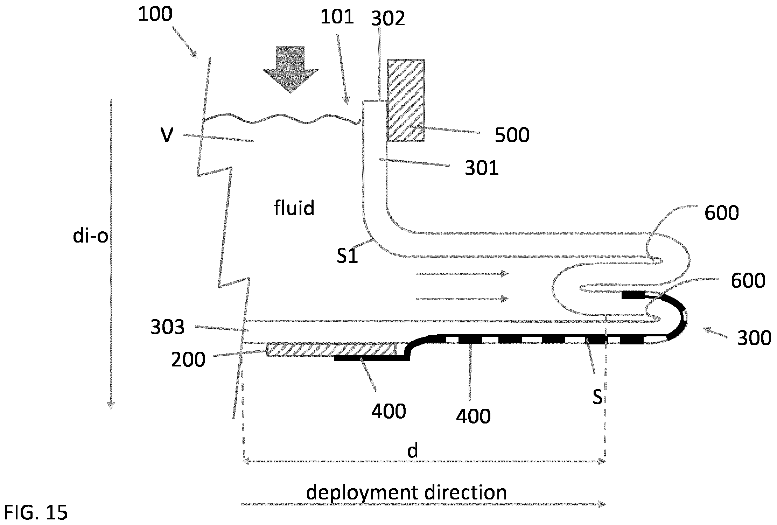

In **Fig. 15** a possible configuration of the device in an intermediate deployed state is shown. In this configuration, the situation is similar to the rest state of the device, except for the fact that the shape of the electrode structure loop is such that a portion of the conductive tracks 400 becomes exposed outside the lumen of the burr hole of diameter d.

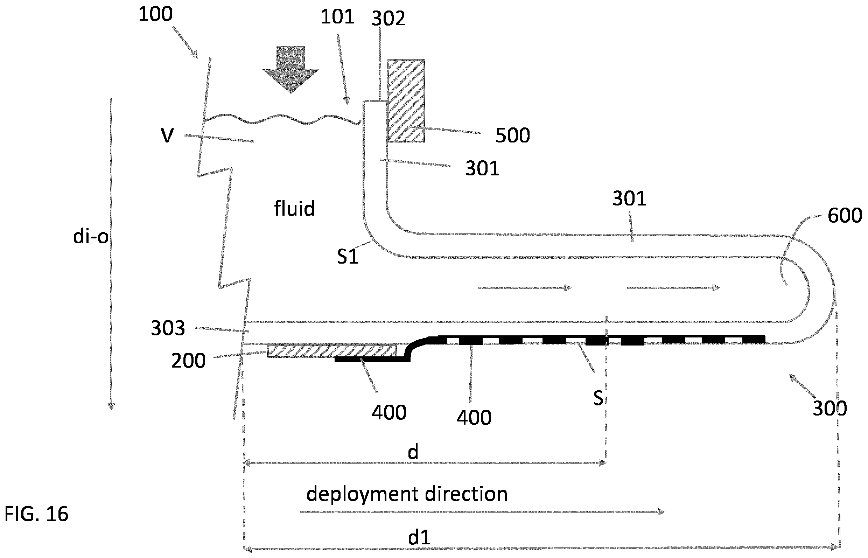

In the fully deployed state, the device is fully deployed, meaning that the eversion event took place. An exemplary eversion event is shown in **Figs. 16****.** In order to operate the eversion of the electrode structure 300, a fluidic pressure is applied inside the reservoir 100, and the electrode structure 300 is released outside the lumen of the burr hole 100 in an inside-out configuration compared to the configuration at the rest state. The above-referenced fluidic pressure may be applied inside the reservoir 100 by, without limitation, a liquid injection inside the reservoir 100 through its inlet 101.

As shown in **Figs. 15** **and** **16****,** upon application of a fluidic pressure inside the reservoir 100, the pockets 600 inside the reservoir start to push the electrode structure 300 so to "unroll" it in a (deployment) direction dp distal compared to the inlet 101 - the eversion event has started. The process proceeds until the entirety of the electrode structure 300 is released outside, the conductive tracks 400 are completely unfolded extended towards the environment to measure and the two fluidic pockets 600 (chambers) are merged into a single pocket together with the main chamber. In the case of a plurality of electrode structures 300, the same event takes place by distributing the fluidic forces among the various branches (the electrode structures).

In the fully deployed state, the device exposes the electrode structure 300 in a configuration that permits the functional and physical contact with target biological surfaces. Particularly, in the fully deployed state, the device exposes the electrode structure 300 so that the entire surface thereof that includes the conductive tracks 400 becomes unfolded extended.

Also, in the fully deployed state, the electrode structure 300 acquires a three-dimensional configuration composed of a border (substantially the support 301) and an inner volume composed of the fluid inflated or injected inside the device. Thus, in the fully deployed state, the electrode structure 300 may resemble to a "tentacle" or a patch with for instance a circular, oval, square or rectangular cross-section, having a length L and a width W typically comprised between 1 mm up to several centimeters (for instance, up to 20 cm), and a height H (the cross-section) typically comprised between 0.2 mm to 1 cm, depending on the needs and circumstances, such as the target biological surfaces or the foreseen applications.

The electrode structure 300 can be rationally designed to have many different shapes, such as branches, tentacles, round or squared patches and so forth, typically depending on the application. For instance, it may be envisageable to design the electrode structure 300 as a large patch, such as a 5 cm x 5 cm patch, to be used as an electrical grid for epilepsy treatment purpose. Therefore, one advantage of the device according to the invention is its universality of design and tailored structure based on the purposed application and the patient it is used on.

In one embodiment, the device may comprise a plurality of reservoirs, each connected with one or more inlets, each reservoir being independently operatively connected with one or more electrode structure. The advantage of this configuration relies in the possibility of timely operate each electrode structure in an independent fashion compared to the other electrode structures. For instance, it may be envisageable to release by eversion a first electrode structure in a first portion of a subject's body part, or to a first portion of a patient's biological surface, in a first time, and a second electrode structure in a second portion of a subject's body part/portion of a patient's biological surface in a second time, by independently inflating each reservoir connected to the relative electrode structure. Additionally or alternatively, it may be envisageable to release by eversion a first electrode structure in a first portion of a subject's body part/portion of a patient's biological surface, in a first time, and a second electrode structure in a second time in the same first portion of a subject's body part/portion of a patient's biological surface. Further, independent electrode structures may exert different functions, such as for instance electrical stimulation of a biological surface and electrical sensing, respectively.

In another additional or alternative embodiment, the electrode structure 300 comprises conductive tracks 400 that are operatively connected with elements such as LEDs. LEDs can be positioned in the device to be at least partially in contact with a patient's biological surface, and can be switched on and off by providing an electrical current through the tracks 400. An advantage of this embodiment relies in the possibility to operate LEDs for e.g. optogenetic experiments or therapy.

With the device of the invention, it is thus possible to contact target biological surfaces in a minimally invasive manner, as the total electrode structure surface is exposed only after eversion, once access to the biological surface has been gained through e.g. small access burr or through holes (such as bores obtainable via laparoscopic surgeries). Advantageously, the contact of target biological surfaces is such that said biological surfaces are contacted with the conductive tracks 400, and at the discretion of the operator (positioning, timing etc).

As it will be evident to a person skilled in the art, in view of the above one aspect of the invention relates to a method for implanting an implantable electrical/electronic biomedical device as disclosed herein, said method comprising the step of:
a) deploying at least one electrode structure deployable by eversion by applying a fluidic pressure inside the reservoir. The deploying step can be carried out by applying a fluidic pressure inside the reservoir via, for instance, a liquid injection.

The device of the invention is intended to be in one embodiment as a peripheral or Central Nervous System (CNS) interface, acting as a bridge between the peripheral/central nervous system and external devices to bidirectionally transducing recorded information and/or sending signals between the human body and a machine processor, and particularly as a fixed or removable implant configured to interface with a biological surface of a subject with the purpose of electrically sensing and/or stimulating an electrical activity in said subject. Accordingly, the implantable electrical/electronic biomedical device of the invention can be configured as a way of example as a fixed or removable neural or nerve implant, heart implant, kidney implant, pancreatic implant, bladder implant, retina implant or gut implant, to mention some.

Accordingly, the method foresees implanting an electrical or electronic biomedical device according to the present disclosure on a biological surface located on a portion of the brain, the heart, the gut, the pancreas, the bladder, a blood vessel, a kidney and/or an eye of said subject in a relatively non-invasive manner.

In one aspect the invention pertains to a system comprising the electric or electronic device as described herein operatively connected with an external device 401 through the at least one conductive track 400. For instance, the system can comprise a device as described herein operatively connected with an external device 401 selected from a non-limiting list comprising an electro-stimulator, an electrical amplification and recording system, a telemetric device, a syringe pump, a pressure based pumping system, a haemostatic pumping system or combinations thereof. The external device 401 can operate in closed-loop or open-loop mode with the electric or electronic device of the invention. The described system can be used e.g. for treating, diagnosing, monitoring and/or preventing signs and/or symptoms associated to pathological conditions.

In view of what said above up to now, it would be evident for a person skilled in the art that in certain embodiments the device of the invention can be used for electrically sensing a physiological parameter or stimulating an electrical response in a biological surface, such as the brain, of a subject. The sensing or stimulation activity can be performed in the frame of a therapeutic or preventive (e.g. for diagnostic purposes) medical treatment in a subject. Accordingly, a further aspect of the invention concerns the use of a device or of a system according to the present disclosure for sensing a physiological signal and/or stimulating an electrical activity and/or pharmacological activity of a biological surface, such as the brain, of a subject. Advantageously, the use of the device or of the system can be intended for treating one or more pathological conditions.

For instance, the device, system and methods according to the present disclosure can be used in a variety of situations for treating one or more pathological conditions wherein sensing and/or electrically stimulating a biological surface of a subject might be beneficial, and in applications related to peripheral nerve pathological conditions including sensory feedback and/or motor feedback.

As a way of example, when in use, the electrode structure 300 is electrically connected through conductors 400 to an external device 401 for delivering an electrical current and/or for biological signal sensing, such as a neuromuscular electrical stimulator or a biological signal acquisition system (e.g. that can acquire for example electromyography -EMG- signals), depending on the application of interest.

The device, system and methods according to the present disclosure can be used and respectively carried out in a variety of situations for treating one or more pathological conditions. A non-limiting, exemplary list of uses of the biomedical device of the present invention comprises retinal, subretinal, and suprachoroidal implants or prosthesis for the treatment of degenerative retinal diseases such as retinitis pigmentosa or age-related macular degeneration (AMD); phrenic nerve stimulators and diaphragm pacemakers to restore breathing function in patients with breathing disorders such as central hypoventilation syndrome (CCHS), central sleep apnea, and diaphragm paralysis; cochlear implants or auditory brainstem implants for the treatment of partial or profound deafness resulting from inner ear damage; stimulation of the brainstem for the treatment of tinnitus; stimulation of the brain for recovery in stroke patients or to treat migraines; electrocorticography (ECoG) to record electrical activity from the cerebral cortex; pacemakers and implantable cardioverter defibrillators; electrical recording for epicardial and endocardial mapping; bladder implants for the treatment of bladder disorders such as detrusor hyperreflexia, detrusor areflexia, overactive bladder syndrome, and urine retention; electrical stimulation of the sphincter for constipation; electrical stimulators for the therapy of pain relief and management thereof, for Parkinson's disease, dystonia or epilepsy; peripheral nerve stimulators for e.g. stimulation of the common peroneal nerve to provide dorsiflexion of the foot and restore muscle movements; lower esophagus stimulators for preventing or treating gastroesophageal reflux; vagal blocking devices for the treatment of obesity by suppressing the sensation of hunger; neuronal interfaces for treating spinal cord injury, strokes or degenerative disorders such as amyotrophic lateral sclerosis cerebral palsy, muscular dystrophy and chronic neuropathic pain, and the like.

The entire system may of course, and preferably, comprise a data processing apparatus operatively connected with the system, the data processing apparatus having a processor comprising instructions configured to operate the system to perform a method according to the invention. The data processing apparatus of the invention can comprise any suitable device such as computers, smartphones, tablets, voice-activated devices (i.e. smart speakers/voice assistants) and the like.

In one embodiment, the data processing apparatus comprises memory storing software modules that provide functionality when executed by the processor. The modules include an operating system that provides operating system functionality for the apparatus. The system, in embodiments that transmit and/or receive data from remote sources, may further include a communication device, such as a network interface card, to provide mobile wireless communication, such as Bluetooth, infrared, radio, Wi-Fi, cellular network, or other next-generation wireless-data network communication. In other embodiments, communication device provides a wired network connection, such as an Ethernet connection or a modem.

Still another aspect of the invention relates to a non-transitory computer readable medium containing a set of instructions that, when executed by data processing apparatus of the system of the invention, cause said data processing apparatus to operate the system to perform a method according to the invention. Further, one aspect of the invention relates to a data processing apparatus comprising the non-transitory computer readable medium of the invention.

In embodiments, the instructions contained by the non-transitory computer readable medium comprise, among others and in any combination:
- instructions for operating an external device 401 according to the present disclosure for delivering an electrical current and/or for biological signal sensing;
- instructions for operating an external device 401 according to the present disclosure to apply a fluidic pressure inside the reservoir 100;
- instructions for operating sensors operatively connected with the device of the invention, as will be detailed hereinafter.

Advantageously, the data processing apparatus may comprise instructions configured to operate an external device 401 according to the present disclosure to apply a fluidic pressure inside the reservoir 100 to perform the eversion process.

As anticipated, in some embodiments the system further comprises one or more sensors operatively connected with the data processing apparatus and with other portions of the system such the conductive tracks/electrodes 400, a portion of the electrode structure 300, such as a portion of the elastic polymeric support 301, or the mechanical support 200, as well as with one or more external devices 401, depending on the needs and circumstances, the sensors being configured for instance to measure, detect and/or analyze a parameter of the system and/or of the surrounding environment.
A "sensor" as used herein is a device that detects (and possibly responds to) signals, stimuli or changes in quantitative and/or qualitative features of a given system, or the environment in general, and provides a corresponding output. The output is generally a signal that is converted to human-readable display at the sensor location or transmitted electronically over a network for reading or further processing. The specific input could be for instance light, heat, motion, moisture, pressure, or any one of a great number of other environmental phenomena. According to the invention, a sensor preferably comprises means for detecting and possibly storing a system's parameter, an environmental parameter or a combination thereof. The sensor can therefore comprise in embodiments a data storage device to hold information, process information, or both. A sensor according to the present disclosure may be for instance a position sensor, an optical sensor such as a light sensor, an infrared sensor or a camera (such as a miniaturized endoscopic camera), a motion sensor, a strain sensor, a touch sensor, a proximity sensor, a temperature sensor, a force / torque sensor, as well as combinations thereof. A strain sensor for instance can be implemented to sense the fluidic pressure differential upon the application of a fluidic pressure inside the reservoir for the deployment of the at least one electrode structure by eversion. The electrode structure growth stops as the fluidic pressure becomes smaller than the friction and until the pressure differential increases sufficiently enough to reiterate an eversion process. The highest pressure differential for deployment remains constant for a given leg cross-section (mm² range) independently on its length, which is one of the main advantages of the pressure-driven eversion.

Sensors may further comprise means for transmitting the detected and possibly stored data concerning the above-mentioned parameters to the data processing apparatus, in some embodiments through a wireless connection. In one embodiment, sensors further comprise means for wirelessly receiving a feedback input from a computer able to regulate the functioning of the system.

An exemplary, non-limiting implemented embodiment of the inventive concept of the invention is provided herein after.

### EXAMPLES

### Design, Principe, and Fabrication

A combination of several key technologies brings together the actuation mechanism, strain sensor, and neural electrodes into a single deployable ECoG system (**Fig.1A**). Two planar elastomeric membranes, the bottom one carrying the electrodes and sensors, are stacked to form the implantable interface. The first membrane hosts microelectrode arrays (reference *34*) prepared with the Silicone-on-silicon process. Metallic interconnects (**Fig.1B-iii**) are thermally evaporated (35-nm-thick gold thin film) on top of the elastomer surface and form elastic conductors that sustain stretch > 30 % strain without electrical failure (references *34, 38*). Electrodes are defined with a Platinum (Pt)-PDMS composite coating that establishes an intimate contact with the nervous tissue, supports low electrical impedance, and are mechanically compliant (**Fig.1B-ii**) (reference *39*). Resistive strain sensors are prepared with similar microcracked gold thin film on the elastomeric substrate. The second membrane of plain silicone completes the stack. A thin flexible printed circuit board is interfaced with the interconnects to form a low geometrical profile connector. Detailed fabrication process is available in the method section (**Fig. 6**). For instance, with reference to **Fig. 1****,** the device including the elastic polymeric support comprises at least two planar elastomeric membranes 1m, 2m stacked one to the other, wherein a first one 1m of the at least two planar elastomeric membranes supports the conductive tracks and defines the surface S of the elastic polymeric support, and a second one 2m of the at least two planar elastomeric membranes defines an opposite surface S1 of the elastic polymeric support arranged to be faced to a fluid in the reservoir, for deployment. **Fig.1B** shows a photograph of a ECoG system deployable by eversion hosting six actuated legs (each corresponding to an electrode structure 300 according to the present disclosure) (6 mm wide and 0.4 mm thick) (**Fig. 1B-i**). Each leg contains four interconnects and associated microelectrodes surrounded by a strain sensor that encircles the outer contour of the deployable strip. In preparation for surgical implantation, the deployable ECoG system is secured to a loader connector using a thin layer of Vinylsiloxane silicone elastomer providing temporary sealing between the array and the loader (**Fig. 7**). Biocompatible aqueous solution (i.e., saline, phosphate buffer solution (PBS), or Dextran solution) is used as a lubricant to reduce layer friction inside the deployable ECoG system during folding and deployment.

For instance, still with reference to **Fig. 1****,** the elastic polymeric support includes a third planar elastomeric membrane 3m arranged between the first and the second planar elastomeric membranes 1m, 2m, the third planar elastomeric membrane 3m supports the at least one sensor. However, nothing prevents that the third planar elastomeric membrane 3m is omitted and that the sensor is directly arranged on the first elastomeric membrane 1m, for instance. In this case, the sensor may be arranged to have a same shape of contour of the first elastomeric membrane. In case a third planar elastomeric membrane 3m is adopted, the at least one sensor is arranged on the third planar elastomeric membrane so as a projection thereof on the first planar elastomeric membrane surrounds the conductive tracks.

As shown in **fig. 1****,** each one of the first to third planar elastomeric surfaces 1m, 2m (in case, 3m) may include a plurality of legs. Each leg of the first planar elastomeric membrane 1m includes conductive tracks and each leg of the third planar elastomeric membrane includes at least one sensor. The at least one sensor is arranged to surround the conductive tracks of the leg of the first planar elastomeric membrane. The legs are curved. However, according to different embodiment, the legs may be straight or curved, preferably tapered.

Next, the deployable ECoG legs are folded into the 20 mm diameter loader (**Fig.1C-E**) that matches typical 20 mm burr holes. The rigid loader is positioned on the cranium, immediately above the preliminary drilled circular burr hole. **Fig.1E-i** shows the 3-step deployment process within a hydrogel phantom human brain (Agarose, 0.5 wt %) and a transparent plastic (PET) skull. A positive fluidic pressure differential is applied inside the loader, increasing the fluidic force *F* at the everting tip (**Fig.1C-i**). The array stays folded until the fluidic force overcomes friction *f* with respect to the increase in the positive fluidic pressure differential. When the fluidic force becomes greater than the friction, the folded array is gently pushed out from the tip via eversion by flipping inside-out and extends under the stiff skull (**Fig. 1C-ii**). As the eversion creates new volume to lengthen the array from the tip, the fluidic pressure differential drops. The tip growth stops as the fluidic pressure becomes smaller than the friction and until the pressure differential increases sufficiently enough to reiterate the above cycle. Note that the highest pressure differential for deployment remains constant for a given leg cross-section (mm² range) independently on its length, which is one of the main advantages of the pressure-driven eversion.

A mechanical support made of 50 µm thick polyimide (PI) film prevents the circular PDMS diaphragm at the center of the deployable ECoG system from inflating and compressing the brain by bulging out with the positive pressure differential. **Fig. 1D-ii** shows a 40 mm diameter, fully deployed implantable system. During deployment, which typically lasts a few tens of seconds, the inflated legs can slightly push against the brain. Resulting compressive strains are discussed in the next section.

Once the system is fully deployed and the lubricant removed, the thin array nicely conforms the surface topology of the brain.

### Structural Characterization

Implementing pressure-driven eversion within the epi-or subdural cortical space calls for minimal compression of the underlying brain tissue. To do so, the inventors first investigated the shape and geometrical parameters of deployable legs, namely the leg width *w*, thickness *h* on straight single legs (**Fig. 2A- i**), the taper angle *a* on tapered legs (**Fig. 2A-ii**), and the radius of curvature *r_{C}* on curved legs (**Fig. 2A-iii**).

Deployment characterization is performed using compressed air to readily monitor the air pressure differential *Pa* inside structural prototypes as it gradually increases until fully deployment then suddenly pressure drops. The inventors defined the metric *Pd,* for deployment pressure, as the maximum of air pressure differential, to quantitatively evaluate the deployability of each design. The highest *Pd* indicates a most difficult design to deploy. **Fig. 2C-i-ii** exemplifies the deployment of two distinct leg geometries with the tapered and curved configuration.

The width and thickness, *w* and *h,* of straight legs significantly affect *Pd* during deployment **(****Fig. 2D-i****):** *Pd* increases as the leg narrows and thickens. Interestingly, tapering or curving affect little *Pd* and the leg deploy-ability (**Fig**. **2D-ii-iii**). Increasing the curvature of the legs is of particular interest as larger surface coverage may be reached (**Fig. 2A-iii**). *P_{d}* stays nearly constant, at 10.4 kPa on average regardless of *r_{C}* ranging from 6 mm to 30 mm, showing that *r_{C}* does not have any substantial effect (**Fig. 2D-iii**).

In the z-direction, i.e. the epidural space, each leg inflates upon pressurization. The leg cross-section initially forms an oval owing to its rectangular relaxed shape. Such inflation can locally and momentarily compress the underlying brain. To quantify the resulting displacements, inventors measured the local strain maps in mock-up models using a digital image correlation (DIC) technique and computed them using finite element modeling (**Fig. 3**).

The soft planar leg transforms into a 3D cylindrical tube with a cross-section that varies from an oval to a circle with respect to the increase in the deployment pressure *Pd* (**Fig. 8A**). Circumferential elongation of the leg envelop is negligible until the cross-section reaches to the circular shape (**Fig.8A-iii**). If *Pd* further increases (Fig. **8A-iii**), following Hooke's law, the leg radially expands with a circumferential stretch that is inversely proportional to the elastic modulus *E* of the PDMS elastomer (**Fig. 8A-iv**). The inventors however did not observe such large radial expansion (**Fig. 8B**) for legs with *h* < 0.4 mm and 4 mm <*w* < 8 mm.

To monitor displacement maps upon pressurization, the inventors modelled the brain and skull using a soft hydrogel (Agarose, 0.5 wt %, *E* ~ 5.3 kPa (reference *40*)) and stiff plastic frame respectively, (**Fig. 3A****)** separated by a 1 mm gap. The gel is coated with randomly dispersed graphene nanoplatelets to allow for DIC tracking. A straight leg prototype (*h* = 0.4 mm and *a* = 0 deg) is inserted in the gap and pressurized with a deployment pressure *Pd* corresponding to its *w* and *h* (**Fig. 2D-ii**). Position of the carbon suspension is tracked with a camera and is processed using an open source DIC analysis software (reference *41*) to reconstruct full field maps of vertical displacement of the hydrogel block (**Fig. 3B**). The inventors defined the resulting indentation *dᵢ* as the vertical displacement of the graphene nanoplatelets from their reference configuration (before the hydrogel is deformed, dashed line in **Fig**. **3A**), with 0 defined at the bottom center of the inflated leg in contact with the hydrogel block as shown **Fig. 3A** and **Fig. 3B****-i.** Inventors also developed an FEM model simulating the similar displacements (**Fig. 3C**) to the experimental ones.

**Fig. 3D** displays *dᵢ* as a function of the hydrogel depth (*x = y =* 0, z) and leg width (*w* = 5 to 8 mm). Gel displacement is the highest (*d*ₘₐₓ) in the immediate vicinity of the leg implant and increases further with wider legs. Beyond 6 mm depth, the observed displacement is below 0.6 mm. **Fig. 3E** shows that the average *d*ₘₐₓ increases with w from 1.7 mm (*w* = 5 mm) to 2.5 mm (*w* = 8 mm), although its slope becomes smaller with respect to the increase in leg width *w*. This may be explained as wider *w* legs may be deployed with a smaller *Pd* thereby inducing lower compression on the brain phantom. Experiments and simulations are in good agreement (**Fig. 3E****),** with overall deviation of only 0.06 mm from the average of the measured *d*_{max.} Thinning the implanted leg reduces further the indentation on the brain (**Fig. 3F**).

This FEM model informs on *Pd* when the implantable system is deployed in an unconstrained environment. The computed values are however underestimated as factors such as poor lubrication, a reduced gap between the brain and the skull, adhesion between the skull and the dura mater in case of epidural implantation, or high curvature of the skull will increase deployment pressure (colored solid lines in **Fig. 3F****).**

### Proof-of-Concept

Next, the inventors implemented the deployable ECoG system *in-vivo* to record cortical brain activity acutely in a minipig model. A prototype of a 6 mm wide, 0.4 mm thick, and 14 mm long single-legged straight *D*-ECoG was developed to be deployed on the somatosensory cortex. Photographs of the folded (pre-implantation) and deployed implant are shown **Fig. 4A****.** The deployable system hosts a 3x4 electrode array (0.3 mm in diameter and 1.5 mm center-to-center pitch) with interconnects and a strain sensor made of microcracked gold thin film (**Fig. 4A-iii**). The strain sensor surrounds the array and allows real-time monitoring of the leg status. First, the array is folded within the holder using a small plastic rod: the initial resistance *R_{O}* of the strain sensor (in its flat, as prepared form) is approximately 10 kΩ then increases up to several mega-ohms upon folding **(****Fig. 4B**). The resistance after folding *Rf* remains in the MΩ range during deployment (increasing *Pa*) and recovers a low value once the array lays (**Fig. 4C**, *R_{d}* < 15 kΩ at *t* > 27 s). Although there are two orders of magnitude difference in electrical track resistance between *Rf* and *Rd*, the resistance after deployment *Rd* remains constant over 50 times of repetitive folding and deployment (**Fig. 4D**), indicating the microcracked gold film is reversibly stretched during folding. Folding and deployment affect little the electrodes' electrochemical impedance spectra of the electrode sites (**Fig. 4E**). Deployment of the leg may be induced using compressed air or a fluid medium (Dextran 15 wt% in this case), which results in slower deployment speed (**Fig. 9A**). The incompressible aqueous solution prevents building of the pneumatic energy compared to compressed air, which leads to fast and uncontrolled deployment (**Fig. 9B**).

### In vivo validation

The inventors showcased *in vivo* deployment and recording capability of the soft system in an anesthetized Göttingen minipig model. The latter presents a large gyrencephalic brain with a vascular density and skull thickness similar than of humans (reference *42*). We record somatosensory evoked potentials (SSEPs) at the surface of the brain in response to the electrical stimulation of the snout.

A single-legged, straight *D*-ECoG strip (**Fig. 4A**) is deployed subdurally at the surface of the brain targeting the frontal lobe and the somatosensory region. The dimensions of the soft implant support a deployment from the superior part of the skull thus do not require a craniotomy above the central sinus vein or displacement of the temporal muscle laterally. Following durotomy, (**Fig. 5A****-i,** see Methods for details), the *D*-ECoG loader is placed at the surface of the brain. The *D*-ECoG is filled beforehand with PBS and is ready to be deployed. The bottom base of the device is placed in contact with the cortex, at the frontal edge of the craniotomy with the folded leg facing to the anterior part as shown **Fig. 5A****-ii.** A syringe pump is used to inject PBS into the loader to initiate and control the subdural deployment. The strain sensor output indicates when the soft leg is fully deployed (*R_{d}* = 21 kΩ at*t* = 42 s, **Fig. 5B**). Next, the loader is removed, and the implant is electrically connected to the electrophysiology recording system.

The inventors recorded evoked potentials from the somatosensory cortex induced by electrical stimulation of the snout delivered at three distinct locations of the snout and two stimulation amplitudes (**Fig. 5C- i**). Averaged signals from selected channels present clear depolarization and polarization peaks (**Fig. 5C-iii**) and a timing similar to previous reports for SSEPs (*43*). The SSEPs with amplitudes reaching >30µV peak amplitude display location-specific characteristics of amplitude and waveform that are consistent with a spatial map of the snout representation (reference 37). The evoked potentials are also modulated with the amplitude of the stimulation current. The position of the deployed array on the somatosensory area is confirmed post-mortem (**Fig. 5D-E**) (reference *44*). The soft implant does not present any folded part and conforms well the cortex surface. Furthermore, after whole brain extraction, no visible damage could be located under or in the vicinity of the deployment location (**Fig. 5E**).

### DISCUSSION

Currently, mechanically passive ECoG grids are used in conjunction with extensive craniotomy that exposes the cortex over areas typically at least as large as the size of the neural interface. Various adverse effects can be associated with large craniotomies including high risk of infection, possible brain damage as well as aesthetic impairments of remaining large scars. These risks significantly limit the adoption and deployment of ECoG arrays for large-scale functional brain mapping or permanent cortical implants for brain-computer interfaces-based therapies. The soft robotic ECoG system of the present disclosure offers an opportunity to access to the very cortical surface of the brain in a minimally invasive manner.

However, the brain is a challenging environment for conventional rigid robots; it is not only extremely soft with Young's modulus of only few kilopascals (references *45, 46*), but also constantly moving inside the skull in accordance with blood pulsation (reference *47*). Any rigid moving parts inside such fragile and dynamic environments cannot only easily damage the delicate brain tissue but can also cause severe foreign body reaction for chronic recording or stimulation (references *5, 48, 49*). The demanding boundary conditions also limit the materials and components available for the implantable robotic devices with a long-term biocompatibility.

Here, various compliant actuators and sensors presented in soft robotics over the past decades provide unique solutions in developing such an implantable 'robot' made of entirely soft materials ensuring high biocompatibility to nervous tissues.

An important consideration in the design of the minimally invasive robotic interface is its insertion through the smallest opening possible for a given number of electrodes. Using the eversion mechanism and an implant design supporting multiple legs, large area (>10 cm²) of the cortex may be interfaced and with a customized electrode size and distribution. With a straight leg design, a significant gap without electrode coverage will lie between the deployed legs. One possible alternative is to curve the electrode strip to cover a larger proportion of the surface in the vicinity of the burr hole. The structural characterization results shows the deployment pressure to evert a pre-folded single *D*-ECoG strip remain constant regardless of the strip's radius of curvature ranging from 6 mm to 30 mm (in **Fig. 2D-iii**). This result suggests one may select an optimized radius of curvature within a large parameter space to satisfy the targeted electrode position and density. The total area of the array coverage is then given by the leg length. The choice of the electrode technology and materials further tailor their dimension and density.

Another way of achieving deployable ECoG system with a high electrode density using eversion is to increase the number of strips connected to the holder thus reduce each leg width. A tradeoff between the deployment pressure, geometry and electrode density is however needed. Narrow leg width increases the deployment pressure (**Fig. 2D-i**). For example, using the soft electrode technology disclosed here, a 5 mm width is the narrowest leg width that can be deployed reliably for a 0.4 mm thick ECoG system (**Fig**. **2D-i**). Reducing the latter's thickness to 0.2 mm decreases by 2.8 times the associated deployment pressure on average (**Fig. 3F**). Thinner implant and narrower leg will also lead to the less indentation of the brain during implantation. Further manufacturing optimization is therefore called upon to reliably produce thin (> 0.2 mm thick) and multi-leg soft implants.

The soft robotic deployable implant implements in situ and real-time monitoring of the expanding legs. This is enabled through thin metallic film strain sensors that are integrated within the process flow of the implantable system. The use of resistance change of conductive elastomer composites or liquid metals has prevailed as strain sensors for the purpose of touch or motion sensing of various wearable and soft robotic devices (references *50-54*). Here the strain sensitivity of thin gold film was leveraged for deployment detection. Inversion or eversion of the *D*-ECoG strip lead significant change in track resistance of the microcracked gold strain sensor (**Fig. 4D**), which nevertheless display reproducible performance (up to 50 reversible loading cycles). The use of a built-in strain sensor also has a distinctive advantage for automation of the deployment system, compared to the use of external visualizing hardware like fluoroscopy. Unlike these imaging tools requiring manual intervention from a human surgeon for controlled deployment, the strain sensor can easily be incorporated into a closed-loop feedback control system to precisely manipulate a pressure source to increase, decrease, or to stop the pressurization of the *D*-ECoG autonomously.

With the present invention, it was shown that by combining soft robotic actuation and soft bioelectronic neurotechnology, one can engineer an implantable neural interface that answer requirements for minimally invasive implantation of large area electrode arrays with soft customized electrode layout.

The concept of leveraging complementary fields of soft robotics and soft bioelectronics presented herein can find applications beyond that of soft neurotechnology (references *55-59*) as the leg and electrode designs may be adapted for specific geometric constraints and targeted organs. For example, deployment over the heart will allow the epicardial mapping of the electrical activity for the diagnosis of arterial fibrillation with much more coverage and precision than current catheter-based solution (reference *60*). Also, the ability to deploy a large electrode array through a small opening can also be leveraged in laparoscopic surgeries in mapping bowel, renal or liver electrical activity (references *61, 62*). Furthermore, spinal cord stimulation electrode arrays (references *27, 63*) can be inserted using a single leg design, while minimizing the size of spinal cord exposure of the conventional open surgery or dual laminectomy (reference *34*). Therefore, the proposed soft robotic neural interfaces will open promising avenues for the development of minimally invasive surface neural interfaces that can significantly improve cost, safety, and usability of various neurotechnology to make it easily accessible in our daily life.

### Materials and Methods

### Study design

The main objective of this study is to combine soft robotic actuation mechanism called 'eversion' and soft bioelectronics to actively deploy soft microelectrode arrays for electrocorticography through a small cranial opening to cover a large area of the brain without causing a damage. This study is designed to achieve the above objective by accomplishing three, non-limiting goals as follows.

The first goal is to establish the design, materials, and manufacturing methods to seamlessly combine all the soft robotic actuation mechanism, strain sensor, and soft microelectrode arrays into a single neural implant with low-profile.

The second goal is to investigate the design rule for optimization of pressure-driven eversion to minimize the indentation of the brain during cortical implantation.

The third goal is to verify the safety and functionality of the proposed soft robotic neural interface via a successful *in-vivo* acute neural recording of a live mini pig's neurophysiological activities.

The deployment pressure was measured five times (*N* = 5) for each *D*-ECoG strip design corresponding to a different combination of design parameters in leg width, thickness, taper angle, and radius of curvature. The indentation of the hydrogel blocks was measured three times (*N* = 3) for each straight *D*-ECoG strips (*h* = 0.4 mm) with four different leg widths (*w* = 5, 6, 7, and 8 mm). The electrochemical impedance spectroscopy of electrode sites and gold interconnects were performed on 10 working electrode sites (*N* = 10). Finally, the measurements of deployment speed were performed five times (*N* = 5) on a straight *D*-ECoG strip (*h* = 0.4 mm, *a* = 6 mm, and *w* = 6 mm) filled with two different fluid media conditions at three different syringe pump speeds.

### Fabrication and experimental methods

Both functional and structural samples of *D*-ECoG were made of medical grade Polydimethylsiloxane (PDMS) elastomer (LSR M130, Elkem Silicones) based on the Silicone-on- Silicon (SoS) process established in our previous work. The *D*-ECoG samples for structural characterizations were fabricated without the interconnects and the Pt-PDMS electrode coatings. According to our experiments, PBS (pH 7.4, Gibco) washes out the Dextran coating inside the *D*- ECoG strip deposited in the process **Fig. 6E** over multiple folding and deployment. The PDMS surface inside the *D*-ECoG tube returns from hydrophilic to hydrophobic that cannot be lubricated with the aqueous solution anymore. Therefore, those structural samples were filled with 15 wt% Dextran solution serving as a lubricant for long-term and repetitive eversion. In the case of the functional *D*-ECoG equipped with electrodes and interconnects for *in-vivo* testing, on the other hand, the sample was filled with PBS to achieve the maximum biocompatibility with the brain tissue for a few times of eversion within a short period.

### Fabrication methods

Fabrication of *D*-ECoG begins with functionalization of a silicon wafer surface used as a supporting carrier. 1H, 1H,2H,2H-perfluorooctyltriethoxysilane (Sigma-Aldrich) is vaporized at room temperature for 30 minutes under weak vacuum with a 4-inch silicon wafer whose surface is activated with O₂ plasma (30W at 0.4 mBar for 30 seconds) (**Fig. 6A**). This creates a hydrophobic monolayer that allows Polydimethylsiloxane (PDMS) elastomer to be released from the silicon wafer surface. A 200-µm-thick PDMS layer (LSR M130, Elkem Silicones) is coated on the wafer surface as the top layer of the *D*-ECoG using a manual film coater (ZUA 2000, Zehntner GmbH) and is cured at 75°C for 3 hours in a convection oven (**Fig. 6A**). A 23-µm-thick polyethylene terephthalate (PET) film is laminated on the top layer as a shadow mask (**Fig. 6A**). Using a femtosecond excimer laser (WS Turret, Optec S.A.), the 11.0 mm in diameter center hole is cut through the top layer, while the air pocket pattern is only cut the 23-µm-thick PET film (**Fig. 6B**). The PET film with air pocket pattern is selectively delaminated, and the exposed PDMS surface of the top layer is activated with O₂ plasma (30W at 0.4 mBar for 30 seconds) (**Fig. 6C**). A sacrificial layer (Dextran solution, 15 wt%) is spin-coated at 2000 rpm on the plasma-activated top layer followed by a drying process for 5 minutes at room temperature (**Fig. 6D**). After removing the entire PET shadow mask from the top layer, a 100- µm-thick PDMS (LSR M130, Elkem Silicones) is blade-coated as the bottom layer of the *D*-ECoG and cured at 75°C for 3 hours in the convection oven, followed by additional hard baking at 110°C for 3 hours in the convection oven to enhance the bonding strength between the top and bottom layers (**Fig. 6E**). Another 23-µm-thick PET film is laminated on top of the bottom layer and is cut out into the 200-µm-wide interconnects and strain sensor patterns using the femtosecond laser machining. The PET films cut into the interconnects and strain sensor are selectively removed from the bottom layer to create a PET-made negative shadow mask pattern (**Fig. 6F**). 5-nm-thick chromium (Cr) followed by 35-nm-thick gold (Au) is deposited on the bottom layer covered with the shadow mask using a thermal evaporation system (Auto 306, Edwards) (**Fig. 6G**). The 40-nm-thick Cr/Au on the PDMS surface shows micro-crack networks after cooled down to room temperature allowing the thin film to stretch on the PDMS surface without electrical failure.

Meanwhile, A 100-µm-thick PDMS (LSR M130, Elkem Silicones) is coated onto a 50-µm-thick PET film using the manual film coater separately. After fully cured at 75°C for 3 hours in the convection oven, the 23-µm-thick PET film is laminated on the PDMS surface. The 300 µm in diameter electrode patterns and FPCB connection opening are cut through the 100-µm-thick PDMS covered with two PET films by the femtosecond excimer laser (**Fig. 6H**). After removing the 23-µm-thick PET films on both side of the PDMS layers, one is on the bottom layer and the other is on 50-µm-thick PET film, the exposed PDMS surfaces are activated with O₂ plasma (100W at 0.2 mBar for 30 seconds) and bonded at room temperature, encapsulating the micro-cracked Cr/Au with the 100-µm-thick PDMS layer (**Fig. 6I**). The 300 µm in diameter electrode cites and the comb-shaped FPCB connection opening on the encapsulation layer are activated with O₂ plasma (20W at 0.6 mBar for 30 seconds) and filled with platinum (Pt)-PDMS composite coatings prepared in accordance with our previous works (**Fig. 6J**) (*39*). Here, the 50-µm-thick PET film serves as a shadow mask for the Pt-PDMS composite coating process. An FPCB is brought down to contact with the Pt-PDMS composite inside the FPCB connection opening, establishing electrical connection with the Cr/Au interconnects. After removing the 50-µm-thick PET shadow mask, the FPCB connection opening is passivated with a room-temperature-vulcanizing (RTV) sealant (Dowsil^{™} 734, Dow Silicones Corp.), providing electrical and mechanical protection (**Fig. 6K**). The Pt-PDMS composite is first cured overnight at room temperature, followed by additional curing at 55°C for 4 hours in the convection oven. The 400 µm in total thickness PDMS elastomer (top, bottom, and encapsulation layers) is cut into the shape of leg-shaped electrode arrays using the femtosecond excimer laser (**Fig. 6L**). The leg-shaped electrode arrays are peeled off from the functionalized wafer surface and are gently washed with deionized water to shed the dust off the device surface after the laser machining (**Fig. 6L**). The Dextran layer between the top and bottom layers is dissolved in deionized water to create the air pocket (**Fig. 6M**).

For the final assembly, a mechanical support is made of a 50-µm-thick polyimide (PI) film by the laser machining, which prevents the bottom layer underneath the 11.0 mm in diameter center hole from bulging out and compressing the brain with a positive pressure differential during deployment (**Fig. 6M**). The mechanical support is attached on the bottom layer using the RTV sealant (Dowsil^{™} 734, Dow Silicones Corp.). The center cylinder (13.0 mm in inner diameter with 1.0 mm in wall thickness) is molded with the same PDMS elastomer (LSR M130, Elkem Silicones) and is cured at 75°C for 3 hours in a convection oven. The center cylinder is manually aligned with the center hole and is attached onto the top layer using a RTV sealant (ADH1 4200, Elkem Silicones) (**Fig. 6N**). Finally, the 3D-printed loader connector (VeroClear^{™}, Stratasys, Ltd.) is assembled with the center cylinder using the RTV sealant (Dowsil^{™} 734, Dow Silicones Corp.) (**Fig. 6N**). In the case of loader system, all individual parts are made of the transparent plastic material (VeroClear^{™}, Stratasys, Ltd.) and are assembled with an adhesive (Loctite^{®} 401^{™}, Henkel Corp.) (**Fig. 7**).

### Experimental Methods

### 1. Experimental set-up and sequence

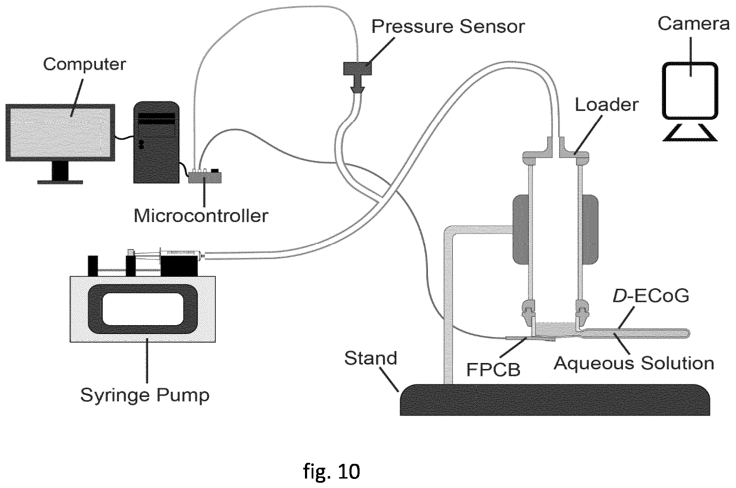

**Fig. 10** shows a schematic image of the experimental set-up for structural characterization as well as track resistance change measurement of a *D*-ECoG strip. First, the *D*-ECoG is filled with aqueous solution as a lubricant, either phosphate buffered saline (PBS, pH 7.4, Gibco) or Dextran (15wt%) depending on the repetition of eversion. The *D*-ECoG is pneumatically connected to the loader using Vinylsiloxane (Flexitime^{®} Correct Flow, Kulzer GmbH) as a sealant. The loader is mounted to a stand to keep a stable position during experiments. In case of using compressed air as the fluid media for eversion, one of two fluidic ports on the loader is connected to a syringe pump (Model '11' 55-4154, Harvard Apparatus) and a pressure sensor (SSCDRRN005PDAA5, Honeywell International Inc.) via silicone tubing (1.6 mm in inner diameter). In this case, the other port on the loader is closed. If the experiment is associated with measurement of the track resistance change, the FPCB on the *D*-ECoG is connected to a voltage divider circuit with a 5.1 kΩ reference resistance. The pressure sensor and the output of voltage divider are connected to a microcontroller (Nano, Arduino) whose voltage readings are recorded using a customized LabVIEW code from a computer. For folding, the *D*-ECoG is inflated with 3 kPa of a positive pressure differential (refer to **Fig. 4B**) to pneumatically stiffen the elastomer strip so that it does not buckle during the folding process. After lubricating the *D*-ECoG surface with ethanol, a plastic rod (1.2 mm in diameter) is used to push the tip of the inflated *D*-ECoG to invert the tube-like structure into the loader. Once the folding is complete, the 3 kPa of pressure differential is removed and the plastic rod is pulled out. During eversion, the syringe pump is infused with a target pumping speed ranging from 40 mL/hr to 240 mL/hr, while the everting *D*-ECoG strip is visually recorded with a digital camera.

In case of using the aqueous solution as the fluid media for eversion (refer to purple bar graphs in **Fig. 4F**), experimental sequence until the folding is performed the same with using the air as the eversion media mentioned above. After the *D*-ECoG is folded, the entire fluidic system including the *D-*ECoG, loader, syringe pump and silicone tubing is filled with either PBS or Dextran (15wt%) using the other port on the loader as an air ventilation port. In this case, the pressure sensor cannot be connected to the fluidic system. Once the whole fluidic system is filled with the aqueous solution, the ventilation port is sealed, and the syringe pump is infused with a designated pumping speed to evert the *D*-ECoG strip.

### 2. Fabrication of hydrogel brain phantom

Agarose powder (Agarose DNA Grade (100 bp - 23 kb), Electran for electrophoresis) was diluted with de-ionized (DI) water (0.5wt%) while heated on a hot plate until the solution became clear. The agarose solution was poured into a negative mold and cooled down until the hydrogel was fully solidified. The fabricated hydrogel phantom was kept in a refrigerator at 3°C overnight before used.

In case of the hydrogel blocks used for the indentation test in **Fig. 3****,** top surface of the hydrogel block was coated with graphene nanoplatelets (5 µm particle size, Sigma-Aldrich). To be specific, the graphene nanoplatelets were mixed with N-Hexane (1wt%, Carlo Erba Reagents S.A.S) and sonicated for 30 min. The solution was well stirred before use and sprayed onto the surface of the agarose block using an air brush (Iwata HP-BH, ANEST Iwata-Medea, Inc.) at approximately 0.5 ~ 1.0 Bar of air pressure (KDG 1000, KVT-Fastening AG). Few droplets of the agarose solution were placed on a flat glass. The graphene coating on top of the hydrogel block was brought down to the glass plate with the uncured agarose drops that create a thin hydrogel encapsulating layer. Due to the difference in surface chemistry, the hydrophobic graphene nanoplatelets were randomly dispersed on the thin hydrophilic agarose layer before it was fully cured. The fabricated agarose blocks were submerged in DI water and stored in a refrigerator at 3°C.

### 3. FEM simulation of indentation of the hydrogel brain phantom

4-node bilinear, hybrid with constant pressure (CPE4H) plane strain elements modeled the cross sectional 2D deployment structure, placed at the 1mm gap between the skull and the brain. Y- symmetry condition at the top layer of the skull and the bottom layer of the brain was applied to fix each coordinate, and X-symmetry condition at the central vertical line was applied to obtain an ideal maximum deformation in the brain from the pressurized deployable strip without uneven distortion.

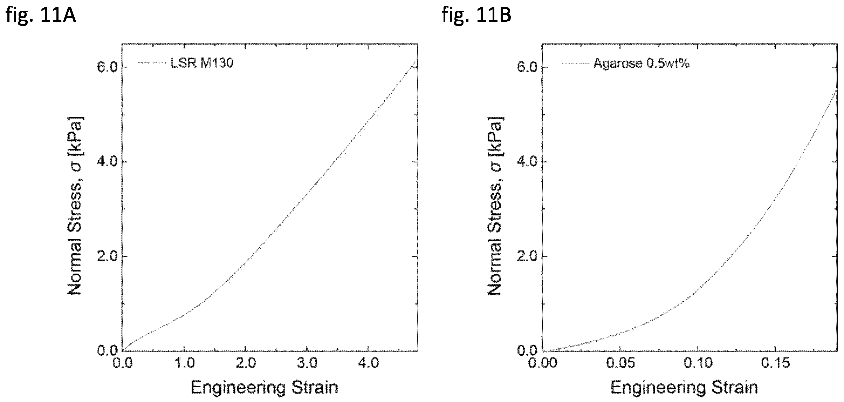

Identical dimensions used in the experimental setup and elastic-plastic material properties of the deployment structure (LSR M130, Elkem Silicones), skull (hard plastic), and brain phantom (hydrogel, agarose 0.5 wt%) were implemented, assuming the 2^{nd} order Ogden for the deployable strip and the 2^{nd} order polynomial hyper elastic behavior for the brain phantom, respectively. The straight, single legged *D*-ECoG strip varied in thickness between 0.1 mm and 0.2 mm and in width from 5 mm to 8 mm with 1 mm of interval. Refined mesh with mesh convergence verified the computational precision using a commercial software application (ABAQUS). The evaluation result of experimented stress-strain data for hyper elastic properties of the M130 (**Fig. 11A**) and the brain phantom (**Fig. 11B**) gave the relevant material parameter as follows; m1 = 0.332, m2 = 0.00939, *a*1 = 2.90, *a*2 = 1.98 for M130 and C10 = 0.000608, C20 = 0.0170 for the brain phantom in MPa. The hard plastic material parameters used for skull were *E* = 10 GPa, *v* = 0.3.

### 4. Electrochemical characterization of Pt-PDMS electrodes/Gold interconnects

A commercial Potentiostat/Galvanostat (Autolab PGSTAT128N, Metrohm AG) was used for activation of electrode coatings and for electrochemical impedance spectroscopy (EIS). First, a *D*- ECoG sample and 5-cm-long platinum (Pt) wire serving as a counter electrode were submerged to phosphate buffered saline (PBS, pH 7.4, Gibco) and connected to the Potentiostat/Galvanostat. A cyclic voltage excitation whose amplitude is ±1 V was applied for 3 cycles on all the *D*-ECoG sample's electrodes to activate Pt-PDMS composite coating. Next, an Ag/AgCl (Metrohm, El. Ag/AgCl DJ RN SC: KCI) reference electrode was submerged in the PBS along with the *D*-ECoG and the Pt electrode and connected to the Potentiostat/Galvanostat in a 3-electrode configuration as shown in **Fig. 12****.** Magnitude and phase of electrochemical impedance on each electrode were measured by a frequency sweep ranging from 1 kHz to 500 kHz.

## Claims

1. An implantable electrical/electronic biomedical device comprising:
a) a reservoir (100) comprising an inlet (101) at a first end and a mechanical support (200) at a second, opposed end, and
b) at least one electrode structure (300) deployable by eversion, said electrode structure (300) comprising:
i) an elastic polymeric support (301) operatively connected to the reservoir's inlet (101) through a first end (302) and to the mechanical support (200) at a second end (303), and
ii) at least one conductive track (400) disposed on a surface (S) of said elastic polymeric support (301).

2. The device of claim 1, wherein said at least one conductive track (400) is compliant.

3. The device of claims 1 or 2, comprising a plurality of electrode structures (300) deployable by eversion.

4. The device of claims 1 to 3, wherein said at least one conductive track (400) is a microelectrode array.

5. The implantable electrical/electronic biomedical device according to claim 1 wherein the reservoir (100) includes a fluid and the at least one electrode structure (300) includes an opposite surface (S1), opposed to said surface (S), faced to the fluid.

6. The implantable electrical/electronic biomedical device according to claim 1 wherein a direction of deployment (dp) of the electrode structure (300) is curved or straight.

7. The implantable electrical/electronic biomedical device according to claim 6 wherein said direction of deployment (dp) lays on a plane perpendicular to a direction (di-o) between the inlet (101) and the mechanical support (200).

8. The implantable electrical/electronic biomedical device according to claim 6 wherein the surface (S) on which the conductive track (400) is arranged is not faced to the fluid.

9. The implantable electrical/electronic biomedical device according to claim 1 wherein said electrode structure (300) forms part of the reservoir (100) and connects the inlet (101) to the mechanical support (200).

10. The implantable electrical/electronic biomedical device according to claim 1 wherein the elastic polymeric support (301) is operatively connected to the reservoir's inlet (101) through the first end (302) fixed to a connecting structure (500), wherein the width of the inlet (101) corresponds to a width of an opening of the connecting structure (500).

11. The implantable electrical/electronic biomedical device according to claim 1 wherein the electrode structure (300) includes at least three folding lines (L) before deployment.

12. The device of claim 3 wherein the plurality of electrode structures (300) are arranged in the reservoir (100) to be deployed by eversion through a burr hole in a skull of a patient, each one of the plurality of electrode structures (300) covering a predetermined length (d1) in respective directions of deployment (dp) when deployed.

13. The device of claim 1 wherein the elastic polymeric support (301) is reversibly stretchable.

14. The device of claim 1 wherein at least some of the conductive tracks (400) are operatively connected with elements such as lighting elements, preferably LEDs.

15. The device of claim 1 wherein at least some of the conductive tracks (400) and/or a portion of the electrode structure (300) and/or a portion of the elastic polymeric support (301) and/or the mechanical support (200) is operatively connected with at least one sensor configured to measure, detect and/or analyze at least one parameter.

16. A data processing apparatus comprising instructions configured to operate an external device (401) to apply a fluidic pressure inside the reservoir (100) according to claim 15 to perform eversion, wherein the data processing apparatus is configured to receive the at least one measured, detected or analyzed parameter from the at least one sensor.

## Patentansprüche

1. Eine implantierbare elektrische/elektronische biomedizinische Vorrichtung, die Folgendes umfassend:
a) ein Reservoir (100), das einen Einlass (101) an einem ersten Ende und eine mechanische Stütze (200) an einem zweiten, gegenüberliegenden Ende umfasst, und
b) mindestens eine Elektrodenstruktur (300), die durch Eversion entfaltbar sind, wobei die Elektrodenstruktur (300) Folgendes umfassend:
i) eine elastische polymere Stütze (301), die über ein erstes Ende (302) mit dem Einlass (101) des Reservoirs und über ein zweites Ende (303) mit der mechanischen Stütze (200) wirkverbunden ist, und
ii) mindestens eine leitfähige Bahn (400), die auf einer Oberfläche (S) der elastischen polymeren Stütze (301) angeordnet ist.

2. Vorrichtung nach Anspruch 1, wobei die mindestens eine leitfähige Bahn (400) nachgiebig ist.

3. Vorrichtung nach Anspruch 1 oder 2, die eine Vielzahl von Elektrodenstrukturen (300) umfasst, die durch Eversion entfaltbar sind.

4. Vorrichtung nach Anspruch 1 bis 3, wobei die mindestens eine leitfähige Bahn (400) eine Mikroelektrodenanordnung ist.

5. Die implantierbare elektrische/elektronische biomedizinische Vorrichtung nach Anspruch 1, wobei das Reservoir (100) eine Flüssigkeit enthält und die mindestens eine Elektrodenstruktur (300) eine der Oberfläche (S) gegenüberliegende Oberfläche (S1) enthält, die der Flüssigkeit zugewandt ist.

6. Die implantierbare elektrische/elektronische biomedizinische Vorrichtung nach Anspruch 1, wobei eine Ausdehnungsrichtung (dp) der Elektrodenstruktur (300) gekrümmt oder gerade ist.

7. Die implantierbare elektrische/elektronische biomedizinische Vorrichtung nach Anspruch 6, wobei die Ausdehnungsrichtung (dp) auf einer Ebene liegt, die senkrecht zu einer Richtung (di-0) zwischen dem Einlass (101) und der mechanischen Stütze (200) ist.

8. Die implantierbare elektrische/elektronische biomedizinische Vorrichtung nach Anspruch 6, wobei die Oberfläche (S), auf der die leitfähige Bahn (400) angeordnet ist, nicht dem Fluid zugewandt ist.

9. Die implantierbare elektrische/elektronische biomedizinische Vorrichtung nach Anspruch 1, wobei die Elektrodenstruktur (300) einen Teil des Reservoirs (100) bildet und den Einlass (101) mit der mechanischen Stütze (200) verbindet.

10. Die implantierbare elektrische/elektronische biomedizinische Vorrichtung nach Anspruch 1, wobei die elastische polymere Stütze (301) über das erste Ende (302), das an einer Verbindungsstruktur (500) befestigt ist, mit dem Einlass (101) des Reservoirs wirkverbunden ist, wobei die Weite des Einlasses (101) der Weite einer Öffnung der Verbindungsstruktur (500) entspricht.

11. Die implantierbare elektrische/elektronische biomedizinische Vorrichtung nach Anspruch 1, wobei die Elektrodenstruktur (300) vor dem Einsatz mindestens drei Faltlinien (L) umfasst.

12. Vorrichtung nach Anspruch 3, wobei die Vielzahl von Elektrodenstrukturen (300) in dem Reservoir (100) angeordnet sind, um durch Eversion durch ein Bohrloch in einem Schädel eines Patienten entfaltet zu werden, wobei jede der Vielzahl von Elektrodenstrukturen (300) eine vorbestimmte Länge (d1) in jeweiligen Ausdehnungsrichtungen (dp) abdeckt, wenn sie entfaltet ist.

13. Vorrichtung nach Anspruch 1, wobei die elastische polymere Stütze (301) reversibel dehnbar ist.

14. Vorrichtung nach Anspruch 1, wobei mindestens einige der leitfähigen Bahnen (400) mit Elementen wie Beleuchtungselementen, vorzugsweise LEDs, wirkverbunden sind.

15. Vorrichtung nach Anspruch 1, wobei mindestens einige der leitfähigen Bahnen (400) und/oder ein Teil der Elektrodenstruktur (300) und/oder ein Teil der elastischen polymeren Stütze (301) und/oder der mechanischen Stütze (200) mit mindestens einem Sensor wirkverbunden ist, der so konfiguriert ist, mindestens einen Parameter zu messen, zu erfassen und/oder zu analysieren.

16. Datenverarbeitungsgerät mit Anweisungen, die konfiguriert sind, eine externe Vorrichtung (401) zu betreiben, um einen Fluiddruck im Inneren des Reservoirs (100) gemäß Anspruch 15 bereitzustellen, um eine Eversion durchzuführen, wobei das Datenverarbeitungsgerät so konfiguriert ist, dass es den mindestens einen gemessenen, erfassten oder analysierten Parameter von dem mindestens einen Sensor empfängt.

## Revendications

1. Dispositif biomédical électrique/électronique implantable comprenant :
a) un réservoir (100) comprenant une entrée (101) à une première extrémité et un support mécanique (200) à une seconde, extrémité opposée, et
b) au moins une structure d'électrode (300) déployable par éversion, ladite structure d'électrode (300) comprenant :
i) un support polymère élastique (301) connecté de manière fonctionnelle à l'entrée (101) du réservoir par une première extrémité (302) et au support mécanique (200) par une seconde extrémité (303), et
ii) au moins une piste conductrice (400) disposée sur une surface (S) dudit support polymère élastique (301).

2. Dispositif selon la revendication 1, dans lequel ladite au moins une piste conductrice (400) est conforme.

3. Dispositif selon les revendications 1 ou 2, comprenant une pluralité de structures d'électrodes (300) déployables par éversion.

4. Dispositif selon les revendications 1 à 3, dans lequel ladite au moins une piste conductrice (400) est un réseau de microélectrodes.

5. Dispositif biomédical électrique/électronique implantable selon la revendication 1, dans lequel le réservoir (100) comprend un fluide et ladite au moins une structure d'électrode (300) comprend une surface opposée (S1), opposée à ladite surface (S), orientée vers le fluide.

6. Dispositif biomédical électrique/électronique implantable selon la revendication 1, dans lequel une direction de déploiement (dp) de la structure d'électrode (300) est courbe ou droite.

7. Dispositif biomédical électrique/électronique implantable selon la revendication 6, dans lequel ladite direction de déploiement (dp) se situe dans un plan perpendiculaire à une direction (di-o) entre l'entrée (101) et le support mécanique (200).

8. Dispositif biomédical électrique/électronique implantable selon la revendication 6, dans lequel la surface (S) sur laquelle est disposée la piste conductrice (400) n'est pas orientée vers le fluide.

9. Dispositif biomédical électrique/électronique implantable selon la revendication 1, dans lequel ladite structure d'électrode (300) fait partie du réservoir (100) et connecte l'entrée (101) au support mécanique (200).

10. Dispositif biomédical électrique/électronique implantable selon la revendication 1, dans lequel le support polymère élastique (301) est connecté de manière fonctionnelle à l'entrée (101) du réservoir par la première extrémité (302) fixée à une structure de connexion (500), dans lequel la largeur de l'entrée (101) correspond à une largeur d'une ouverture de la structure de connexion (500).

11. Dispositif biomédical électrique/électronique implantable selon la revendication 1, dans lequel la structure d'électrode (300) comprend au moins trois lignes de pliage (L) avant le déploiement.

12. Dispositif selon la revendication 3, dans lequel la pluralité de structures d'électrodes (300) est disposée dans le réservoir (100) pour être déployée par éversion à travers un trou de trépanation dans le crâne d'un patient, chacune des structures d'électrodes (300) couvrant une longueur prédéterminée (d1) dans les directions respectives de déploiement (dp) lorsqu'elles sont déployées.

13. Dispositif selon la revendication 1, dans lequel le support polymère élastique (301) est extensible réversiblement.

14. Dispositif selon la revendication 1, dans lequel au moins certaines des pistes conductrices (400) sont connectées de manière fonctionnelle à des éléments tels que des éléments d'éclairage, de préférence des DEL.

15. Dispositif selon la revendication 1, dans lequel au moins certaines des pistes conductrices (400) et/ou une partie de la structure d'électrode (300) et/ou une partie du support polymère élastique (301) et/ou le support mécanique (200) sont connectés de manière fonctionnelle à au moins un capteur configuré pour mesurer, détecter et/ou analyser au moins un paramètre.

16. Appareil de traitement de données comprenant des instructions configurées pour faire fonctionner un dispositif externe (401) afin d'appliquer une pression fluidique à l'intérieur du réservoir (100) selon la revendication 15, pour réaliser une éversion, dans lequel l'appareil de traitement de données est configuré pour recevoir l'au moins un paramètre mesuré, détecté ou analysé à partir de l'au moins au capteur.
